(19) Europäisches Patentamt
European Patent Office
Office européen des brevets

(11) EP 1 287 133 B1

(12) EUROPEAN PATENT SPECIFICATION

(45) Date of publication and mention of the grant of the patent:
**13.12.2006 Bulletin 2006/50**

(21) Application number: **01945139.2**

(22) Date of filing: **16.05.2001**

(51) Int Cl.:
*C12N 15/12* (2006.01) *C07K 14/705* (2006.01)
*C12N 1/21* (2006.01) *C12N 5/10* (2006.01)
*C12Q 1/68* (2006.01) *A61K 48/00* (2006.01)
*C12N 15/62* (2006.01) *G01N 33/68* (2006.01)
*A61K 31/7068* (2006.01) *A61K 39/395* (2006.01)

(86) International application number:
**PCT/EP2001/005559**

(87) International publication number:
**WO 2001/087929 (22.11.2001 Gazette 2001/47)**

# (54) REGULATION OF HUMAN DOPAMINE-LIKE G PROTEIN-COUPLED RECEPTOR

REGULATION EINES MENSCHLICHEN DOPAMINE-ÄHNLICHEN G-PROTEIN GEKOPPELTEN REZEPTOR.

REGULATION DE RECEPTEUR COUPLE AUX PROTEINES G DE TYPE DOPAMINE HUMAIN

(84) Designated Contracting States:
**AT BE CH CY DE DK ES FI FR GB GR IE IT LI LU MC NL PT SE TR**

(30) Priority: **18.05.2000 US 205195 P**

(43) Date of publication of application:
**05.03.2003 Bulletin 2003/10**

(73) Proprietor: **Bayer HealthCare AG**
**51368 Leverkusen (DE)**

(72) Inventor: **RAMAKRISHNAN, Shyam**
**Brighton, MA 02135 (US)**

(56) References cited:
**EP-A- 1 092 727** **WO-A-00/22131**
**WO-A-00/31258** **WO-A-00/50562**
**WO-A-01/32864** **WO-A-01/36473**
**WO-A-01/42288**

- **DATABASE EMBL [Online] HS20I3 accession number AL035423, 12 February 1999 (1999-02-12) A. PEARCE: "HUMAN DNA SEQUENCE FROM CLONE 20I3 ON CHROMOSOME XQ25-26 CONTAINS A GENE FOR BRAIN MITOCHONDRIAL CARRIER PROTEIN-1 (BMCP1), ESTS, STSS, GSSS AND A CPG ISLAND" XP002166396**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

SEQUENCE LISTING

<110> Bayer AG

<120> REGULATION OF HUMAN DOPAMINE-LIKE
G PROTEIN-COUPLED RECEPTOR

<130> REGULATION OF HUMAN DOPAMINE-LIKE

<140>
<141>

<150> 60/205,195
<151> 2000-05-18

<160> 3

<170> Patent In Ver. 2.0

<210> 1
<211> 1008
<212> DNA
<213> Human

<400> 1

```
atggaatcat ctttctcatt tggagtgatc cttgctgtcc tggcctccct catcattgct 60
actaacacac tagtggctgt ggctgtgctg ctgttgatcc acaagaatga tggtgtcagt 120
ctctgcttca ccttgaatct ggctgtggct gacaccttga ttggtgtggc catctctggc 180
ctactcacag accagctctc cagcccttct cggcccacac agaagaccct gtgcagcctg 240
cggatggcat ttgtcacttc ctccgcagct gcctctgtcc tcacggtcat gctgatcacc 300
tttgacaggt accttgccat caagcagccc ttccgctact tgaagatcat gagtgggttc 360
gtggccgggg cctgcattgc cgggctgtgg ttagtgtctt acctcattgg cttcctccca 420
ctcggaatcc ccatgttcca gcagactgcc tacaaagggc agtgcagctt ctttgctgta 480
tttcaccctc acttcgtgct gaccctctcc tgcgttggct tcttcccagc catgctcctc 540
tttgtcttct tctactgcga catgctcaag attgcctcca tgcacagcca gcagattcga 600
aagatggaac atgcaggagc catggctgga ggttatcgat ccccacggac tcccagcgac 660
ttcaaagctc tccgtactgt gtctgttctc attgggagct ttgctctatc ctggaccccc 720
ttccttatca ctggcattgt gcaggtggcc tgccaggagt gtcacctcta cctagtgctg 780
gaacggtacc tgtggctgct cggcgtgggc aactccctgc tcaacccact catctatgcc 840
tattggcaga aggaggtgcg actgcagctc taccacatgg ccctaggagt gaagaaggtg 900
ctcacctcat tcctcctctt tctctcggcc aggaattgtg gcccagagag gcccagggaa 960
agttcctgtc acatcgtcac tatctccagc tcagagtttg atggctaa             1008
```

<210> 2
<211> 335
<212> PRT
<213> Human

<400> 2

Met Glu Ser Ser Phe Ser Phe Gly Val Ile Leu Ala Val Leu Ala Ser
1 5 10 15

Leu Ile Ile Ala Thr Asn Thr Leu Val Ala Val Ala Val Leu Leu Leu
20 25 30

Ile His Lys Asn Asp Gly Val Ser Leu Cys Phe Thr Leu Asn Leu Ala
35 40 45

Val Ala Asp Thr Leu Ile Gly Val Ala Ile Ser Gly Leu Leu Thr Asp
50 55 60

Gln Leu Ser Ser Pro Ser Arg Pro Thr Gln Lys Thr Leu Cys Ser Leu
65 70 75 80

Arg Met Ala Phe Val Thr Ser Ser Ala Ala Ala Ser Val Leu Thr Val
85 90 95

Met Leu Ile Thr Phe Asp Arg Tyr Leu Ala Ile Lys Gln Pro Phe Arg
100 105 110

Tyr Leu Lys Ile Met Ser Gly Phe Val Ala Gly Ala Cys Ile Ala Gly
115 120 125

Leu Trp Leu Val Ser Tyr Leu Ile Gly Phe Leu Pro Leu Gly Ile Pro
130 135 140

Met Phe Gln Gln Thr Ala Tyr Lys Gly Gln Cys Ser Phe Phe Ala Val
145 150 155 160

Phe His Pro His Phe Val Leu Thr Leu Ser Cys Val Gly Phe Phe Pro
165 170 175

Ala Met Leu Leu Phe Val Phe Phe Tyr Cys Asp Met Leu Lys Ile Ala
180 185 190

Ser Met His Ser Gln Gln Ile Arg Lys Met Glu His Ala Gly Ala Met
195 200 205

Ala Gly Gly Tyr Arg Ser Pro Arg Thr Pro Ser Asp Phe Lys Ala Leu
210 215 220

Arg Thr Val Ser Val Leu Ile Gly Ser Phe Ala Leu Ser Trp Thr Pro
225 230 235 240

Phe Leu Ile Thr Gly Ile Val Gln Val Ala Cys Gln Glu Cys His Leu
245 250 255

Tyr Leu Val Leu Glu Arg Tyr Leu Trp Leu Leu Gly Val Gly Asn Ser
260 265 270

Leu Leu Asn Pro Leu Ile Tyr Ala Tyr Trp Gln Lys Glu Val Arg Leu
275 280 285

Gln Leu Tyr His Met Ala Leu Gly Val Lys Lys Val Leu Thr Ser Phe
290 295 300

Leu Leu Phe Leu Ser Ala Arg Asn Cys Gly Pro Glu Arg Pro Arg Glu
305 310 315 320

Ser Ser Cys His Ile Val Thr Ile Ser Ser Ser Glu Phe Asp Gly
325 330 335

<210> 3
<211> 451
<212> PRT
<213> Human

<400> 3

```
Met Tyr Thr Pro His Pro Phe Gly Phe Leu Ile Ile Leu Val Pro Met
 1               5                  10                  15

Thr Asn Ala Met Arg Ala Ile Ala Ala Ile Ala Ala Gly Val Gly Ser
            20                  25                  30

Val Ala Ala Thr Val Ala Thr Ser Thr Thr Ser Ser Ile Ser Ser Ser
        35                  40                  45

Thr Thr Ile Ile Asn Thr Ser Ser Ala Thr Thr Ile Gly Gly Asn His
    50                  55                  60

Thr Ser Gly Ser Thr Gly Phe Ser Thr Asn Ser Thr Leu Leu Asp Ala
65                  70                  75                  80

Asp His Leu Pro Leu Gln Leu Thr Thr Ala Lys Val Asp Leu Asp Ile
                85                  90                  95

Glu Ile Asp Ile Gln Leu Leu Thr Asn Gly Tyr Asp Gly Thr Thr Leu
            100                 105                 110

Thr Ser Phe Tyr Asn Glu Ser Ser Trp Thr Asn Ala Ser Glu Met Asp
        115                 120                 125

Thr Ile Val Gly Glu Glu Pro Glu Pro Leu Ser Leu Val Ser Ile Val
```

130 135 140

Val Val Gly Ile Phe Leu Ser Val Leu Ile Phe Leu Ser Val Ala Gly
145 150 155 160

Asn Ile Leu Val Cys Leu Ala Ile Tyr Thr Asp Gly Ser Leu Pro Arg
165 170 175

Ile Gly Asn Leu Phe Leu Ala Ser Leu Ala Ile Ala Asp Leu Phe Val
180 185 190

Ala Ser Leu Val Met Thr Phe Ala Gly Val Asn Asp Leu Leu Gly Tyr
195 200 205

Trp Ile Phe Gly Ala Gln Phe Cys Asp Thr Trp Val Ala Phe Asp Val
210 215 220

Met Cys Ser Thr Ala Ser Ile Leu Asn Leu Cys Ala Ile Ser Met Asp
225 230 235 240

Arg Tyr Ile His Ile Lys Asp Pro Leu Arg Tyr Gly Arg Trp Val Thr
245 250 255

Arg Arg Val Ala Val Ile Thr Ile Ala Ala Ile Trp Leu Leu Ala Ala
260 265 270

Phe Val Ser Phe Val Pro Ile Ser Leu Gly Ile His Arg Pro Asp Gln
275 280 285

Pro Leu Ile Phe Glu Asp Asn Gly Lys Lys Tyr Pro Thr Cys Ala Leu
290 295 300

Asp Leu Thr Pro Thr Tyr Ala Val Val Ser Ser Cys Ile Ser Phe Tyr
305 310 315 320

Phe Pro Cys Val Val Met Ile Gly Ile Tyr Cys Arg Leu Tyr Cys Tyr
325 330 335

Ala Gln Lys His Val Lys Ser Ile Lys Ala Val Thr Arg Pro Gly Glu
340 345 350

Val Ala Glu Lys Gln Arg Tyr Lys Ser Ile Arg Arg Pro Lys Asn Gln
355 360 365

Pro Lys Lys Phe Lys Val Arg Asn Leu His Thr His Ser Ser Pro Tyr
370 375 380

His Val Ser Asp His Lys Ala Ala Val Thr Val Gly Val Ile Met Gly

385 390 395 400

Val Phe Leu Ile Cys Trp Val Pro Phe Phe Cys Val Asn Ile Thr Ala
405 410 415

Ala Phe Cys Lys Ser Asp Arg Phe Ile Thr Asp Tyr Ala Ala Lys Asn
420 425 430

Val Val Val Met Asn Ser Gly Arg Ser Ser Ala Glu Leu Glu Gln Val
435 440 445

Ser Ala Ile
450

## Description

TECHNICAL FIELD OF THE INVENTION

The invention relates to the area of G-protein coupled receptors. More particularly, it relates to the area of human dopamine-like G protein-coupled receptor and its regulation.

BACKGROUND OF THE INVENTION

*G-Protein Coupled Receptors*

Many medically significant biological processes are mediated by signal transduction pathways that involve G-proteins (Lefkowitz, *Nature 351,* 353-354, 1991). The family of G-protein coupled receptors (GPCR) includes receptors for hormones, neurotransmitters, growth factors, and viruses. Specific examples of GPCRs include receptors for such diverse agents as calcitonin, adrenergic hormones, endothelin, cAMP, adenosine, acetylcholine, serotonin, dopamine, histamine, thrombin, kinin, follicle stimulating hormone, opsins, endothelial differentiation gene-1, rhodopsins, odorants, cytomegalovirus, G-proteins themselves, effector proteins such as phospholipase C, adenyl cyclase, and phosphodiesterase, and actuator proteins such as protein kinase A and protein kinase C.

The GPCR protein superfamily now contains over 250 types of paralogues, receptors that represent variants generated by gene duplications (or other processes), as opposed to orthologues, the same receptor from different species. The superfamily can be broken down into five families: Family I, receptors typified by rhodopsin and the β-adrenergic receptor and currently represented by over 200 unique members (reviewed by Dohlman *et al., Ann. Rev. Biochem. 60,* 653-88, 1991, and references therein); Family II, the recently characterized parathyroid hormone/calcitonin/secretin receptor family (Juppner *et al., Science 254,* 1024-26, 1991; Lin *et al., Science 254,* 1022-24, 1991); Family III, the metabotropic glutamate receptor family in mammals (Nakanishi, *Science 258,* 597-603, 1992); Family IV, the cAMP receptor family, important in the chemotaxis and development of *D. discoideum* (Klein *et al., Science 241,* 1467-72, 1988; and Family V, the fingal mating pheromone receptors such as STE2 (reviewed by Kurjan, *Ann. Rev. Biochem. 61,* 1097-1129, 1992).

GPCRs possess seven conserved membrane-spanning domains connecting at least eight divergent hydrophilic loops. GPCRs (also known as 7TM receptors) have been characterized as including these seven conserved hydrophobic stretches of about 20 to 30 amino acids, connecting at least eight divergent hydrophilic loops. Most GPCRs have single conserved cysteine residues in each of the first two extracellular loops, which form disulfide bonds that are believed to stabilize functional protein structure. The seven transmembrane regions are designated as TM1, TM2, TM3, TM4, TM5, TM6, and TM7. TM3 has been implicated in signal transduction.

Phosphorylation and lipidation (palmitylation or famesylation) of cysteine residues can influence signal transduction of some GPCRs. Most GPCRs contain potential phosphorylation sites within the third cytoplasmic loop and/or the carboxy terminus. For several GPCRs, such as the β-adrenergic receptor, phosphorylation by protein kinase A and/or specific receptor kinases mediates receptor desensitization.

For some receptors, the ligand binding sites of GPCRs are believed to comprise hydrophilic sockets formed by several GPCR transmembrane domains. The hydrophilic sockets are surrounded by hydrophobic residues of the GPCRs. The hydrophilic side of each GPCR transmembrane helix is postulated to face inward and form a polar ligand binding site. TM3 has been implicated in several GPCRs as having a ligand binding site, such as the TM3 aspartate residue. TM5 serines, a TM6 asparagine, and TM6 or TM7 phenylalanines or tyrosines also are implicated in ligand binding.

GPCRs are coupled inside the cell by heterotrimeric G-proteins to various intracellular enzymes, ion channels, and transporters (*see* Johnson *et al., Endoc. Rev. 10*, 317-331, 1989). Different G-protein alpha-subunits preferentially stimulate particular effectors to modulate various biological functions in a cell. Phosphorylation of cytoplasmic residues of GPCRs is an important mechanism for the regulation of some GPCRs. For example, in one form of signal transduction, the effect of hormone binding is the activation inside the cell of the enzyme, adenylate cyclase. Enzyme activation by hormones is dependent on the presence of the nucleotide GTP. GTP also influences hormone binding. A G-protein connects the hormone receptor to adenylate cyclase. G-protein exchanges GTP for bound GDP when activated by a hormone receptor. The GTP-carrying form then binds to activated adenylate cyclase. Hydrolysis of GTP to GDP, catalyzed by the G-protein itself, returns the G-protein to its basal, inactive form. Thus, the G-protein serves a dual role, as an intermediate that relays the signal from receptor to effector, and as a clock that controls the duration of the signal.

Over the past 15 years, nearly 350 therapeutic agents targeting GPCRs receptors have been successfully introduced onto the market. This indicates that these receptors have an established, proven history as therapeutic targets. Clearly, there is an ongoing need for identification and characterization of further GPCRs which can play a role in preventing, ameliorating, or correcting dysfunctions or diseases including, but not limited to, infections such as bacterial, fungal, protozoan, and viral infections, particularly those caused by HIV viruses, pain, cancers, anorexia, bulimia, asthma, Parkinson's diseases, acute heart failure, hypotension, hypertension, urinary retention, osteoporosis, angina pectoris, myocardial infarction, ulcers, asthma, allergies, benign prostatic hypertrophy, and psychotic and neurological disorders, including anxiety, schizophrenia, manic depression, delirium, dementia, several mental retardation, and dyskinesias, such as Huntington's disease and Tourett's syndrome.

*Dopamine and Dopamine Receptors*

Dopamine is a neurotransmitter that participates in a variety of different functions mediated by the nervous system, including vision, movement, and behavior (see U.S. Patent 5,883,226 and *Cooper et al.,* 1978, THE BIOCHEMICAL BASIS OF NEUROPHARMACOLOGY, 3d ed., Oxford University Press, New York, pp, 161-195). The diverse physiological actions of dopamine are in turn mediated by its interaction with two of the basic types of G protein-coupled receptors, D1 and D2, which respectively stimulate and inhibit the enzyme adenylyl cyclase (Kebabian & Calne, 1979, *Nature 277,* 93-96). Alterations in the number or activity of these receptors may be a contributory factor in disease states such as Parkinson's disease (a movement disorder) and schizophrenia (a behavioral disorder).

A great deal of information has accumulated on the biochemistry of the D 1 and D2 dopamine receptors, and methods have been developed to solubilize and purify these receptor proteins (see Senogles *et al.,* 1986, *Biochemistry 25,* 749-753; Sengoles *et al.,* 1988, *J. Biol. Chem.* 263, 18996-19002; Gingrich *et al.,* 1988, *Biochemistry 27,* 3907-3912). The D1 dopamine receptor in several tissues appears to be a glycosylated membrane protein of about 72 kD (Amlaiky *et al.,* 1987, *Mol. Pharmacol. 31,* 129-134; Ninik *et al.,* 1988, *Biochemistry 27,* 7594-7599). The D2 receptor has been suggested to have a higher molecular weight of about 90-150 kD (Amlaiky & Caron, 1985, *J. Biol. Chem. 260, 1983-* 1986; Amlaiky & Caron, 1986, *J. Neurochem. 47,* 196-204; Jarvie *et al.,* 1988, *Mol. Pharmacol. 34,* 91-97). Much less is known about a recently discovered additional dopamine receptor, termed D3 (Sokoloff *et al.,* 1990, *Nature 347,* 146-151).

Dopamine receptors are primary targets in the clinical treatment of psychomotor disorders such as Parkinson's disease and affective disorders such as schizophrenia (Seeman *et al.,* 1987, *Neuropsychopharm. 1,* 5-15; Seeman, 1987, *Synapse 1,* 152-333). The three different dopamine receptors (D1, D2, D3) have been cloned as a result of nucleotide sequence homology which exists between these receptor genes (Bunzow *et al.,* 1988, *Nature 336,* 783-787; Grandy *et al.,* 1989, *Proc. Natl. Acad. Sci. USA 86,* 9762-9766; Dal Toso *et al.,* 1989, *EMBO J. 8,* 4025-4034; Zhou *et al.,* 1990, *Nature 346,* 76-80; Sunahara *et al.,* 1990, *Nature 346,* 80-83; Sokoloff *et al.,* 1990, *Nature 347,* 146-151).

The antipsychotic clozapine is useful for socially withdrawn and treatment-resistant schizophrenics (see Kane *et al.,* 1990, *Nature 347,* 146-151), but unlike other antipsychotic drugs, clozapine does not cause tardive dyskinesia (see Casey, 1989, *Psychopharmacology 99,* 547-553). Clozapine, however, has dissociation constants for D2 and D3 which are 3 to 30-fold higher than the therapeutic free concentration of clozapine in plasma water (Ackenheil *et al.,* 1976, *Arzneim-Forsch 26,* 1156-58; Sandoz Canada, Inc., 1990, Clozaril: Summary of preclinical and clinical data). This suggests the existence of dopamine receptors more sensitive to the antipsychotic clozapine than those known in the prior art heretofore.

Because of dopamine's diverse biological effects, there is a need m the art to identify additional members of the dopamine GCPR family whose activity can be regulated to provide therapeutic effects.

WO 0022131 discloses a human orphan GPCR denominated nRUP 3 with its nucleotide and amino acid sequence being identical to SEQ ID NOs: 1 and 2.

SUMMARY OF THE INVENTION

Another embodiment of the invention is a method of screening for agents useful in the treatment of Alzheimer's disease. A test compound is contacted with a dopamine-like GPCR polypeptide comprising an amino acid sequence selected from the group consisting of:

amino acid sequences which are at least 90% identical to the amino acid sequence shown in SEQ ID NO. 2; and

the amino acid sequence shown in SEQ ID NO. 2.

A dopamine-like GPCR activity of the polypeptide is detected. A test compound which decreases dopamine-like GPCR activity of the polypeptide relative to dopamine-like GPCR activity in the absence of the test compound is thereby identified as a potential agent for the treatment of Alzheimer's disease.

The invention thus provides a human dopamine-like G protein coupled receptor which can be used to identify test compounds which may act as antagonists at the receptor site and which are useful in the treatment of Alzheimer's disease. Human dopamine-like G protein-coupled receptor and fragments thereof also are useful in raising specific antibodies which can block the receptor and effectively prevent ligand binding, and which are useful in the treatment of Alzheimer's disease.

BRIEF DESCRIPTION OF THE DRAWING

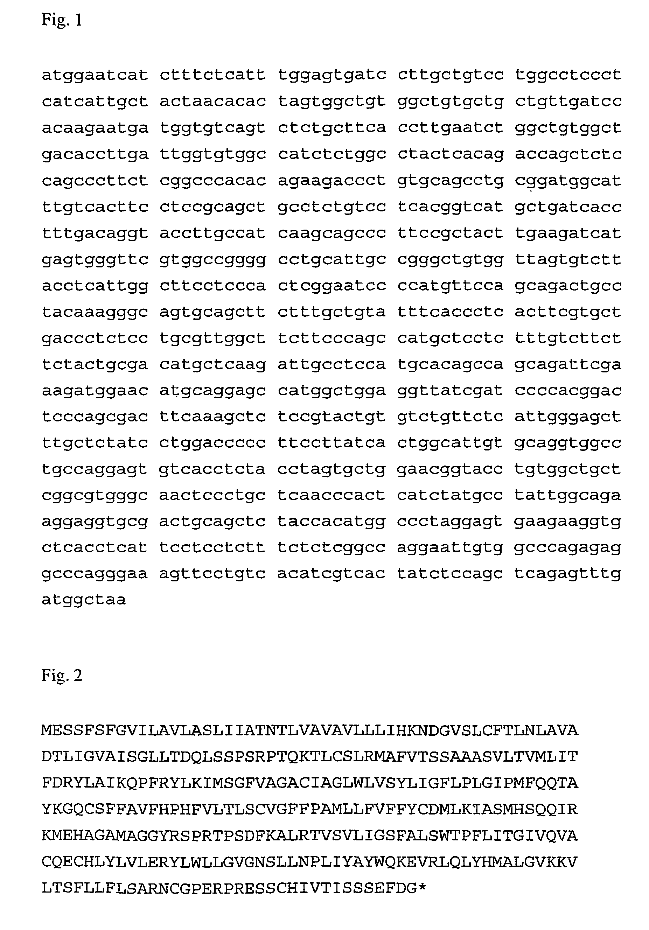

Fig. 1 shows the DNA-sequence encoding a dopamine-like GPCR polypeptide.
Fig. 2 shows the amino acid sequence deduced from the DNA-sequence of Fig.1.

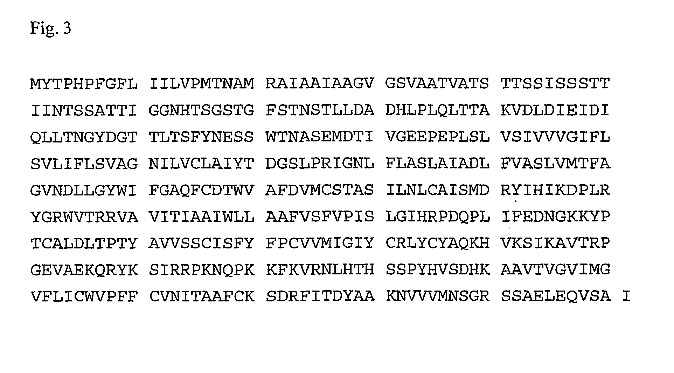

Fig. 3 shows the amino acid sequence of a protein identified with SwissProt Accession No. P41596.

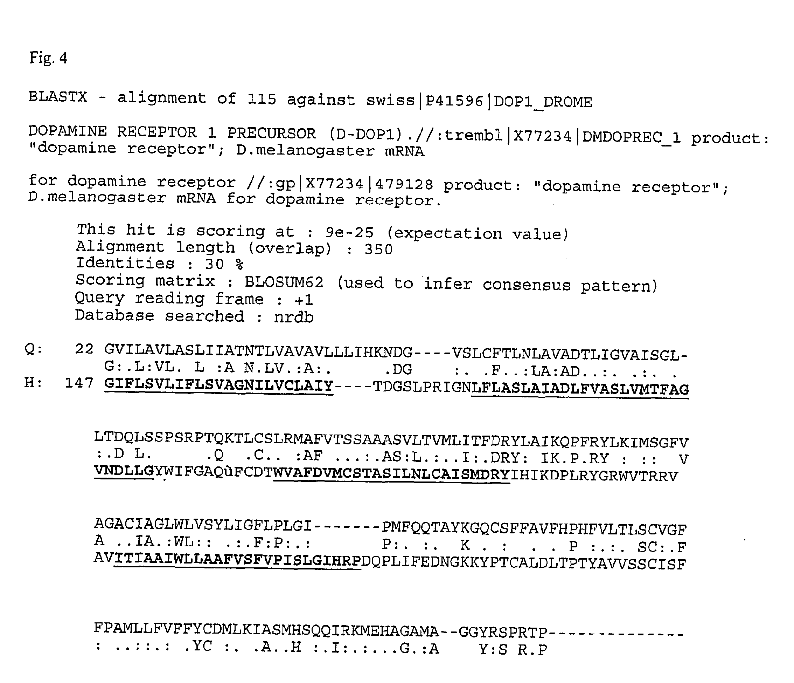

Fig. 4 shows the BLASTX alignment of dopamine-like GPCR polypeptide of Fig.2 with SwissProt Accession No. P41596 of Fig. 3.

DETAILED DESCRIPTION OF THE INVENTION

It has been discovered by the present applicant that a dopamine receptor-like GPCR (DA-like GPCR), particularly a human dopamine receptor-like GPCR, can be used in therapeutic methods, and in screening methods for the identification of antagonits useful to treat disorders such as Alzheimer's disease. Human DA-like GPCR also can be used to screen for human DA-like GPCR antagonists useful for the treatment of Alzheimer's disease.

Human DA-like GPCR is 30% identical over 350 amino acids to the *D. melanogaster* protein identified with SwissProt Accession No. P41596 and annotated as a dopamine 1 receptor precursor. Thus, human DA-like GPCR is expected to be useful for the same purposes as previously identified dopamine receptors. For example, human DA- like GPCR made from cloned genes in accordance with the present invention may be used for screening compounds for DA-like GPCR activity or for determining the amount of a dopaminergic drug in a solution (*e.g.*, blood plasma or serum). Host cells transformed with expression vectors comprising human DA-like polynucleotides can be used to screen compounds for DA-like GPCR binding activity. Competitive binding assays in which such procedures may be carried out are well known, as illustrated by the specific examples below. By selection of host cells which do not ordinarily express a dopamine receptor, preparations free of D1A receptors, D2 receptors, and other dopamine receptor subtypes can be obtained. Further, human DA-like GPCR agonists and antagonists can be identified by transforming host cells which express adenylyl cyclase with expression vectors of the present invention. Membranes obtained from such cells can be used in biochemical studies wherein the activity of the adenylyl cyclase is monitored. DA-like GPCR agonists will stimulate the adenylyl cyclase. Such cells must be capable of operatively associating the DA-like GPCR with the adenylyl cyclase, *i.e.,* G protein must also be present in the cell membranes. Procedures for carrying out assays such as these are also described in greater detail in the examples which follow.

Isolated and purified DA-like GPCRs of the present invention also is DA-like GPCR can be purified from cell membranes or lysed cell fractions containing the receptor, as described below, in accordance with known procedures, including column chromatography (e.g., ion exchange, gel filtration, electrophoresis, affinity chromatography, etc.), optionally followed by crystallization (See generally "Enzyme Purification and Related Techniques," *Methods in Enzymology 22*, 233-577, 1977).

*Polypeptides*

DA-like, GPCR polypeptides comprise at least 20, 30, 40, 50, 53, 78 100, 125, 150, 175, 200, 250, 300, or 350 contiguous amino acids selected from the amino acid sequence shown in SEQ ID NO.2 or a biologically active variant thereof, as defined below. A DA-like GPCR polypeptide therefore can be a portion of a DA-like GPCR protein, a full-length DA-like GPCR protein, or a fusion protein comprising all or a portion of a DA-like GPCR protein. A coding sequence for SEQ ID NO. 2 is shown in SEQ ID NO. 1.

*Biologically Active Variants*

DA-like GPCR polypeptide variants which are biologically active, *i.e.,* retain the ability to bind a dopamine or a dopamine-like ligand to produce a biological effect, such as cyclic AMP formation, mobilization of intracellular calcium, or phosphoinositide metabolism, also are DA-like GPCR polypeptides. Preferably, naturally or non-naturally occurring DA-like GPCR polypeptide variants have amino acid sequences which are at least 90% identical to the amino acid sequence shown in SEQ ID NO. 2. Percent identity between a putative DA-like GPCR polypeptide variant and an amino acid sequence of SEQ ID NO. 2 is determined using the Blast2 alignment program (Blosum62, Expect 10, standard genetic codes).

Variations in percent identity can be due, for example, to amino acid substitutions, insertions, or deletions.

Amino acid substitutions are defined as one for one amino acid replacements. They are conservative in nature when the substituted amino acid has similar structural and/or chemical properties. Examples of conservative replacements are substitution of a leucine with an isoleucine or valine, an aspartate with a glutamate, or a threonine with a serine.

Amino acid insertions or deletions are changes to or within an amino acid sequence. They typically fall in the range of about 1 to 5 amino acids. Guidance in determining which amino acid residues can be substituted, inserted, or deleted without abolishing biological or immunological activity of a DA-like GPCR polypeptide can be found using computer programs well known in the art, such as DNASTAR software. Whether an amino acid change results in a biologically active DA-like GPCR polypeptide can readily be determined by assaying for binding to a ligand or by conducting a functional assay, as described for example, in the specific Examples, below.

*Fusion Proteins*

Fusion proteins are useful for generating antibodies against DA-like GPCR polypeptide amino acid sequences and for use in various assay systems. For example, fusion proteins can be used to identify proteins which interact with portions of a DA-like GPCR polypeptide. Protein affinity chromatography or library-based assays for protein- protein interactions, such as the yeast two-hybrid or phage display systems, can be used for this purpose. Such methods are well known in the art and also can be used as drug screens.

A DA-like GPCR polypeptide fusion protein comprises two polypeptide segments fused together by means of a peptide bond. The first polypeptide segment comprises at least 20, 30, 40, 50, 53, 78 100, 125, 150, 175, 200, 250, 300, or 350 contiguous amino acids of SEQ ID NO. 2 or of a biologically active variant, such as those described above. The first polypeptide segment also can comprise full-length DA-like GPCR protein.

The second polypeptide segment can be a full-length protein or a protein fragment. Proteins commonly used in fusion protein construction include β-galactosidase, β-glucuronidase, green fluorescent protein (GFP), autofluorescent proteins, including blue fluorescent protein (BFP), glutathione-S-transferase (GST), luciferase, horseradish peroxidase (HRP), and chloramphenicol acetyltransferase (CAT). Additionally, epitope tags are used in fusion protein constructions, including histidine (His) tags, FLAG tags, influenza hemagglutinin (HA) tags, Myc tags, VSV-G tags, and thioredoxin (Trx) tags. Other fusion constructions can include maltose binding protein (MBP), S-tag, Lex a DNA binding domain (DBD) fusions, GAL4 DNA binding domain fusions, and herpes simplex virus (HSV) BP16 protein fusions. A fusion protein also can be engineered to contain a cleavage site located between the DA-like GPCR polypeptide-encoding sequence and the heterologous protein sequence, so that the DA-like GPCR polypeptide can be cleaved and purified away from the heterologous moiety.

A fusion protein can be synthesized chemically, as is known in the art. Preferably, a fusion protein is produced by covalently linking two polypeptide segments or by standard procedures in the art of molecular biology. Recombinant DNA methods can be used to prepare fusion proteins, for example, by making a DNA construct which comprises coding sequences selected from SEQ ID NO. 1 in proper reading frame with nucleotides encoding the second polypeptide segment and expressing the DNA construct in a host cell, as is known in the art. Many kits for constructing fusion proteins are available from companies such as Promega Corporation (Madison, WI), Stratagene (La Jolla, CA), CLONTECH (Mountain View, CA), Santa Cruz Biotechnology (Santa Cruz, CA), MBL International Corporation (MIC; Watertown, MA), and Quantum Biotechnologies (Montreal, Canada; 1-888-DNA-KITS).

*Identification of Species Homologs*

Species homologs of human DA-like GPCR polypeptide can be obtained using DA-like GPCR polypeptide polynucleotides (described below) to make suitable probes or primers for screening cDNA expression libraries from other species, such as mice, monkeys, or yeast, identifying cDNAs which encode homologs of DA-like GPCR polypeptide, and expressing the cDNAs as is known in the art.

*Identification of Polynucleotide Variants and Homologs*

Variants and homologs of the DA-GPCR polynucleotides described above also are DA-like GPCR polynucleotides. Typically, homologous DA-like GPCR polynucleotide sequences can be identified by hybridization of candidate polynucleotides to known DA-like GPCR polynucleotides under stringent conditions, as is known in the art. For example, using the following wash conditions--2X SSC (0.3 M NaCl, 0.03 M sodium citrate, pH 7.0), 0.1% SDS, room temperature twice, 30 minutes each; then 2X SSC, 0.1% SDS, 50°C once, 30 minutes; then 2X SSC, room temperature twice, 10 minutes each--homologous sequences can be identified which contain at most about 25-30% basepair mismatches. More preferably, homologous nucleic acid strands contain 15-25% basepair mismatches, even more preferably 5-15% basepair mismatches.

Species homologs of the DA-like GPCR polynucleotides disclosed herein also can be identified by making suitable probes or primers and screening cDNA expression libraries from other species, such as mice, monkeys, or yeast. Human variants of DA-like GPCR polynucleotides can be identified, for example, by screening human cDNA expression libraries. It is well known that the $T_m$ of a double-stranded DNA decreases by 1-1.5°C with every 1% decrease in homology (Bonner *et al., J. Mol. Biol. 81*, 123 (1973). Variants of human DA-like GPCR polynucleotides or DA-GPCR polynucleotides of other species can therefore be identified by hybridizing a putative homologous DA-like GPCR polynucleotide with a polynucleotide having a nucleotide sequence of SEQ ID NO. 1 or the complement thereof to form a test hybrid. The melting temperature of the test hybrid is compared with the melting temperature of a hybrid comprising polynucleotides having perfectly complementary nucleotide sequences, and the number or percent of basepair mismatches within the test hybrid is calculated.

Nucleotide sequences which hybridize to DA-like GPCR polynucleotides or their complements following stringent hybridization and/or wash conditions also are DA-like GPCR polynucleotides. Stringent wash conditions are well known and understood in the art and are disclosed, for example, in Sambrook *et al.,* MOLECULAR CLONING: A LABORATORY MANUAL, 2d ed., 1989, at pages 9.50-9.51.

Typically, for stringent hybridization conditions a combination of temperature and salt concentration should be chosen that is approximately 12-20°C below the calculated $T_m$ of the hybrid under study. The $T_m$ of a hybrid between a DA-like GPCR polynucleotide having a nucleotide sequence shown in SEQ ID NO. 1 or the complement thereof and a polynucleotide sequence which is at least about 50, preferably about 75, 90, 96, or 98% identical to one of those nucleotide sequences can be calculated, for example, using the equation of Bolton and McCarthy, *Proc. Natl. Acad. Sci. U.S.A. 48,* 1390 (1962):

$$T_m = 81.5°C - 16.6(\log_{10}[Na^+]) + 0.41(\%G + C) - 0.63(\%formamide) - 600/l,$$

where $l$ = the length of the hybrid in basepairs.

Stringent wash conditions include, for example, 4X SSC at 65°C, or 50% formamide, 4X SSC at 42°C, or 0.5X SSC, 0.1% SDS at 65°C. Highly stringent wash conditions include, for example, 0.2X SSC at 65°C.

*Preparation of Polynucleotides*

A naturally occurring DA-like GPCR polynucleotide can be isolated free of other cellular components such as membrane components, proteins, and lipids. Polynucleotides can be made by a cell and isolated using standard nucleic acid purification techniques, or synthesized using an amplification technique, such as the polymerase chain reaction (PCR), or by using an automatic synthesizer. Methods for isolating polynucleotides are routine and are known in the art. Any such technique for obtaining a polynucleotide can be used to obtain isolated DA-like GPCR polynucleotides. For example, restriction enzymes and probes can be used to isolate polynucleotide fragments which comprises DA-like GPCR nucleotide sequences. Isolated polynucleotides are in preparations which are free or at least 70, 80, or 90% free of other molecules.

DA-like GPCR cDNA molecules can be made with standard molecular biology techniques, using DA-like GPCR mRNA as a template. DA-like GPCR cDNA molecules can thereafter be replicated using molecular biology techniques known in the art and disclosed in manuals such as Sambrook *et al.* (1989). An amplification technique, such as PCR, can be used to obtain additional copies of polynucleotides of the invention, using either human genomic DNA or cDNA as a template.

Alternatively, synthetic chemistry techniques can be used to synthesizes DA-GPCR polynucleotides. The degeneracy of the genetic code allows alternate nucleotide sequences to be synthesized which will encode a DA-like GPCR polypeptide having, for example, an amino acid sequence shown in SEQ ID NO. 2 or a biologically active variant thereof.

*Extending Polynucleotides*

Various PCR-based methods can be used to extend the nucleic acid sequences disclosed herein to detect upstream sequences such as promoters and regulatory elements. For example, restriction-site PCR uses universal primers to retrieve unknown sequence adjacent to a known locus (Sarkar, *PCR Methods Applic. 2*, 318-322, 1993). Genomic DNA is first amplified in the presence of a primer to a linker sequence and a primer specific to the known region. The amplified sequences are then subjected to a second round of PCR with the same linker primer and another specific primer internal to the first one. Products of each round of PCR are transcribed with an appropriate RNA polymerase and sequenced using reverse transcriptase.

Inverse PCR also can be used to amplify or extend sequences using divergent primers based on a known region (Triglia *et al., Nucleic Acids Res. 16,* 8186, 1988). Primers can be designed using commercially available software, such as OLIGO 4.06 Primer Analysis software (National Biosciences Inc., Plymouth, Minn.), to be 22-30 nucleotides in length, to have a GC content of 50% or more, and to anneal to the target sequence at temperatures about 68-72°C. The method uses several restriction enzymes to generate a suitable fragment in the known region of a gene. The fragment is then circularized by intramolecular ligation and used as a PCR template.

Another method which can be used is capture PCR, which involves PCR amplification of DNA fragments adjacent to a known sequence in human and yeast artificial chromosome DNA (Lagerstrom *et al., PCR Methods Applic. 1,* 111-119, 1991). In this method, multiple restriction enzyme digestions and ligations also can be used to place an engineered double-stranded sequence into an unknown fragment of the DNA molecule before performing PCR.

Another method which can be used to retrieve unknown sequences is that of Parker *et al., Nucleic Acids Res. 19,* 3055-3060, 1991). Additionally, PCR, nested primers, and PROMOTERFINDER libraries (CLONTECH, Palo Alto, Calif.) can be used to walk genomic DNA (CLONTECH, Palo Alto, Calif.). This process avoids the need to screen libraries and is useful in finding intron/exon junctions.

When screening for full-length cDNAs, it is preferable to use libraries that have been size-selected to include larger cDNAs. Randomly-primed libraries are preferable, in that they will contain more sequences which contain the 5' regions of genes. Use of a randomly primed library may be especially preferable for situations in which an oligo d(T) library does not yield a full-length cDNA. Genomic libraries can be useful for extension of sequence into 5' non-transcribed regulatory regions.

Commercially available capillary electrophoresis systems can be used to analyze the size or confirm the nucleotide sequence of PCR or sequencing products. For example, capillary sequencing can employ flowable polymers for electrophoretic separation, four different fluorescent dyes (one for each nucleotide) which are laser activated, and detection of the emitted wavelengths by a charge coupled device camera. Output/light intensity can be converted to electrical signal using appropriate software (*e.g.* GENOTYPER and Sequence NAVIGATOR, Perkin Elmer), and the entire process from loading of samples to computer analysis and electronic data display can be computer controlled. Capillary electrophoresis is especially preferable for the sequencing of small pieces of DNA which might be present in limited amounts in a particular sample.

## *Obtaining Polypeptides*

DA-like GPCR polypeptides can be obtained, for example, by purification from human cells, by expression of DA-like GPCR polynucleotides, or by direct chemical synthesis.

### *Protein Purification*

DA-like GPCR polypeptides can be purified from any human cell which expresses the receptor, including host cells which have been transfected with DA-like GPCR polynucleotides. Melanocytes, fetal heart, and uterus are all particularly useful sources of DA-like GPCR polypeptides. A purified DA-like GPCR polypeptide is separated from other compounds which normally associate with the DA-like GPCR polypeptide in the cell, such as certain proteins, carbohydrates, or lipids, using methods well-known in the art. Such methods include, but are not limited to, size exclusion chromatography, ammonium sulfate fractionation, ion exchange chromatography, affinity chromatography, and preparative gel electrophoresis.

DA-like GPCR polypeptide can be conveniently isolated as a complex with its associated G protein, as described in the specific examples, below. A preparation of purified DA-like GPCR polypeptides is at least 80% pure; preferably, the preparations are 90%, 95%, or 99% pure. Purity of the preparations can be assessed by any means known in the art, such as SDS-polyacrylamide gel electrophoresis.

### *Expression of Polynucleotides*

To express a DA-like GPCR polynucleotide, the polynucleotide can be inserted into an expression vector which contains the necessary elements for the transcription and translation of the inserted coding sequence. Methods which are well known to those skilled in the art can be used to construct expression vectors containing sequences encoding DA-like GPCR polypeptides and appropriate transcriptional and translational control elements. These methods include *in vitro* recombinant DNA techniques, synthetic techniques, and *in vivo* genetic recombination. Such techniques are described, for example, in Sambrook *et al.* (1989) and in Ausubel *et al.,* CURRENT PROTOCOLS IN MOLECULAR BIOLOGY, John Wiley & Sons, New York, N.Y., 1989.

A variety of expression vector/host systems can be utilized to contain and express sequences encoding a DA-like GPCR polypeptide. These include, but are not limited to, microorganisms, such as bacteria transformed with recombinant bacteriophage, plasmid, or cosmid DNA expression vectors; yeast transformed with yeast expression vectors, insect cell systems infected with virus expression vectors (*e.g.*, baculovirus), plant cell systems transformed with virus expression vectors (*e.g.*, cauliflower mosaic virus, CaMV; tobacco mosaic virus, TMV) or with bacterial expression vectors (*e.g.*, Ti or pBR322 plasmids), or animal cell systems.

The control elements or regulatory sequences are those non-translated regions of the vector -- enhancers, promoters, 5' and 3' untranslated regions - which interact with host cellular proteins to carry out transcription and translation. Such elements can vary in their strength and specificity. Depending on the vector system and host utilized, any number of suitable transcription and translation elements, including constitutive and inducible promoters, can be used. For example, when cloning in bacterial systems, inducible promoters such as the hybrid lacZ promoter of the BLUESCRIPT phagemid (Stratagene, LaJolla, Calif.) or pSPORT1 plasmid (Life Technologies) and the like can be used. The baculovirus polyhedrin promoter can be used in insect cells. Promoters or enhancers derived from the genomes of plant cells (*e.g.,* heat shock, RUBISCO, and storage protein genes) or from plant viruses (*e.g.,* viral promoters or leader sequences) can be cloned into the vector. In mammalian cell systems, promoters from mammalian genes or from mammalian viruses are preferable. If it is necessary to generate a cell line that contains multiple copies of a nucleotide sequence encoding a DA-like GPCR polypeptide, vectors based on SV40 or EBV can be used with an appropriate selectable marker.

*Bacterial and Yeast Expression Systems*

In bacterial systems, a number of expression vectors can be selected depending upon the use intended for the DA-like GPCR polypeptide. For example, when a large quantity of a DA-like GPCR polypeptide is needed for the induction of antibodies, vectors which direct high level expression of fusion proteins that are readily purified can be used. Such vectors include, but are not limited to, multifunctional *E. coli* cloning and expression vectors such as BLUESCRIPT (Stratagene). In a BLUESCRIPT vector, a sequence encoding the DA-like GPCR polypeptide can be ligated into the vector in frame with sequences for the amino-terminal Met and the subsequent 7 residues of β-galactosidase so that a hybrid protein is produced. pIN vectors (Van Heeke & Schuster, *J. Biol. Chem. 264,* 5503-5509, 1989) or pGEX vectors (Promega, Madison, Wis.) also can be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. Proteins made in such systems can be designed to include heparin, thrombin, or factor Xa protease cleavage sites so that the cloned polypeptide of interest can be released from the GST moiety at will.

In the yeast *Saccharomyces cerevisiae,* a number of vectors containing constitutive or inducible promoters such as alpha factor, alcohol oxidase, and PGH can be used. For reviews, see Ausubel *et al.* (1989) and Grant *et al., Methods Enzymol. 153,* 516-544, 1987.

*Plant and Insect Expression Systems*

If plant expression vectors are used, the expression of sequences encoding DA-like GPCR polypeptides can be driven by any of a number of promoters. For example, viral promoters such as the 35S and 19S promoters of CaMV can be used alone or in combination with the omega leader sequence from TMV (Takamatsu, *EMBO J. 6,* 307-311, 1987). Alternatively, plant promoters such as the small subunit of RUBISCO or heat shock promoters can be used (Coruzzi *et al., EMBO J. 3,* 1671-1680, 1984; Broglie *et al., Science 224,* 838-843, 1984; Winter *et al., Results Probl. Cell Differ. 17,* 85-105, 1991). These constructs can be introduced into plant cells by direct DNA transformation or by pathogen-mediated transfection. Such techniques are described in a number of generally available reviews (*e.g.*, Hobbs or Murray, in McGRAW HILL YEARBOOK OF SCIENCE AND TECHNOLOGY, McGraw Hill, New York, N.Y., pp. 191-196, 1992).

An insect system also can be used to express a DA-like GPCR polypeptide. For example, in one such system *Autographa californica* nuclear polyhedrosis virus (AcNPV) is used as a vector to express foreign genes in *Spodoptera frugiperda* cells or in *Trichoplusia* larvae. Sequences encoding DA-like GPCR polypeptides can be cloned into a non-essential region of the virus, such as the polyhedrin gene, and placed under control of the polyhedrin promoter. Successful insertion of DA-like GPCR polypeptides will render the polyhedrin gene inactive and produce recombinant virus lacking coat protein. The recombinant viruses can then be used to infect *S. frugiperda cells* or *Trichoplusia* larvae in which DA-like GPCR polypeptides can be expressed (Engelhard *et al., Proc. Nat. Acad. Sci. 91,* 3224-3227, 1994).

*Mammalian Expression Systems*

A number of viral-based expression systems can be used to express DA-like GPCR polypeptides in mammalian host cells. For example, if an adenovirus is used as an expression vector, sequences encoding DA-like GPCR polypeptides can be ligated into an adenovirus transcription/translation complex comprising the late promoter and tripartite leader sequence. Insertion in a non-essential E1 or E3 region of the viral genome can be used to obtain a viable virus which is capable of expressing a DA-like GPCR polypeptide in infected host cells (Logan & Shenk, *Proc. Natl. Acad. Sci. 81,* 3655-3659, 1984). If desired, transcription enhancers, such as the Rous sarcoma virus (RSV) enhancer, can be used to increase expression in mammalian host cells.

Human artificial chromosomes (HACs) also can be used to deliver larger fragments of DNA than can be contained and expressed in a plasmid. HACs of 6M to 10M are constructed and delivered to cells via conventional delivery methods (*e.g.*, liposomes, polycationic amino polymers, or vesicles).

Specific initiation signals also can be used to achieve more efficient translation of sequences encoding DA-like GPCR polypeptides. Such signals include the ATG initiation codon and adjacent sequences. In cases where sequences encoding a DA-like GPCR polypeptide, its initiation codon, and upstream sequences are inserted into the appropriate expression vector, no additional transcriptional or translational control signals may be needed. However, in cases where only coding sequence, or a fragment thereof, is inserted, exogenous translational control signals (including the ATG initiation codon) should be provided. The initiation codon should be in the correct reading frame to ensure translation of the entire insert. Exogenous translational elements and initiation codons can be of various origins, both natural and synthetic. The efficiency of expression can be enhanced by the inclusion of enhancers which are appropriate for the particular cell system which is used (see Scharf *et al., Results Probl. Cell Differ. 20,* 125-162, 1994).

*Host Cells*

A host cell strain can be chosen for its ability to modulate the expression of the inserted sequences or to process the expressed DA-like GPCR polypeptide in the desired fashion. Such modifications of the polypeptide include, but are not limited to, acetylation, carboxylation, glycosylation, phosphorylation, lipidation, and acylation. Post-translational processing which cleaves a "prepro" form of the polypeptide also can be used to facilitate correct insertion, folding and/or function. Different host cells which have specific cellular machinery and characteristic mechanisms for post-translational activities (*e.g.*, CHO, HeLa, MDCK, HEK293, and WI38), are available from the American Type Culture Collection (ATCC; 10801 University Boulevard, Manassas, VA 20110-2209) and can be chosen to ensure the correct modification and processing of the foreign protein.

Stable expression is preferred for long-term, high-yield production of recombinant proteins. For example, cell lines which stably express DA-like GPCR polypeptides can be transformed using expression vectors which can contain viral origins of replication and/or endogenous expression elements and a selectable marker gene on the same or on a separate vector. Following the introduction of the vector, cells can be allowed to grow for 1-2 days in an enriched medium before they are switched to a selective medium. The purpose of the selectable marker is to confer resistance to selection, and its presence allows growth and recovery of cells which successfully express the introduced DA-like GPCR sequences. Resistant clones of stably transformed cells can be proliferated using tissue culture techniques appropriate to the cell type. See, for example, ANIMAL CELL CULTURE, R.I. Freshney, ed., 1986.

Any number of selection systems can be used to recover transformed cell lines.

These include, but are not limited to, the herpes simplex virus thymidine kinase (Wigler *et al., Cell 11*, 223-32, 1977) and adenine phosphoribosyltransferase (Lowy *et al., Cell 22,* 817-23, 1980) genes which can be employed in $tk^-$ or $aprt^-$ cells, respectively. Also, antimetabolite, antibiotic, or herbicide resistance can be used as the basis for selection. For example, *dhfr* confers resistance to methotrexate (Wigler *et al., Proc. Natl. Acad. Sci. 77*, 3567-70, 1980), *npt* confers resistance to the aminoglycosides, neomycin and G-418 (Colbere-Garapin *et al., J. Mol. Biol. 150,* 1-14, 1981), and *als* and *pat* confer resistance to chlorsulfuron and phosphinotricin acetyltransferase, respectively (Murray, 1992, *supra*). Additional selectable genes have been described. For example, *trpB* allows cells to utilize indole in place of tryptophan, or *hisD,* which allows cells to utilize histinol in place of histidine (Hartman & Mulligan, *Proc. Natl. Acad. Sci. 85,* 8047-51, 1988). Visible markers such as anthocyanins, β-glucuronidase and its substrate GUS, and luciferase and its substrate luciferin, can be used to identify transformants and to quantify the amount of transient or stable protein expression attributable to a specific vector system (Rhodes *et al., Methods Mol. Biol. 55,* 121-131, 1995).

*Detecting Expression*

Although the presence of marker gene expression suggests that the DA-like GPCR polynucleotide is also present, its presence and expression may need to be confirmed. For example, if a sequence encoding a DA-like GPCR polypeptide is inserted within a marker gene sequence, transformed cells containing sequences which encode a DA-like GPCR polypeptide can be identified by the absence of marker gene function. Alternatively, a marker gene can be placed in tandem with a sequence encoding a DA-like GPCR polypeptide under the control of a single promoter. Expression of the marker gene in response to induction or selection usually indicates expression of the DA-like GPCR polynucleotide.

Alternatively, host cells which contain a DA-like GPCR polynucleotide and which express a DA-like GPCR polypeptide can be identified by a variety of procedures known to those of skill in the art. These procedures include, but are not limited to, DNA-DNA or DNA-RNA hybridizations and protein bioassay or immunoassay techniques which include membrane, solution, or chip-based technologies for the detection and/or quantification of nucleic acid or protein. For example, the presence of a polynucleotide sequence encoding a DA-like GPCR polypeptide can be detected by DNA-DNA or DNA-RNA hybridization or amplification using probes or fragments or fragments of polynucleotides encoding a DA-like GPCR polypeptide. Nucleic acid amplification-based assays involve the use of oligonucleotides selected from sequences encoding a DA-like GPCR polypeptide to detect transformants which contain a DA-like GPCR polynucleotide.

A variety of protocols for detecting and measuring the expression of a DA-like GPCR polypeptide, using either polyclonal or monoclonal antibodies specific for the polypeptide, are known in the art. Examples include enzyme-linked immunosorbent assay (ELISA), radioimmunoassay (RIA), and fluorescence activated cell sorting (FACS). A two-site, monoclonal-based immunoassay using monoclonal antibodies reactive to two non-interfering epitopes on a DA-like GPCR polypeptide can be used, or a competitive binding assay can be employed. These and other assays are described in Hampton *et al.,* SEROLOGICAL METHODS: A LABORATORY MANUAL, APS Press, St. Paul, Minn., 1990) and Maddox *et al., J. Exp. Med. 158,* 1211-1216, 1983).

A wide variety of labels and conjugation techniques are known by those skilled in the art and can be used in various nucleic acid and amino acid assays. Means for producing labeled hybridization or PCR probes for detecting sequences related to polynucleotides encoding DA-like GPCR polypeptides include oligolabeling, nick translation, end-labeling, or PCR amplification using a labeled nucleotide. Alternatively, sequences encoding a DA-like GPCR polypeptide can be cloned into a vector for the production of an mRNA probe. Such vectors are known in the art, are commercially available, and can be used to synthesize RNA probes *in vitro* by addition of labeled nucleotides and an appropriate RNA polymerase such as T7, T3, or SP6. These procedures can be conducted using a variety of commercially available kits (Amersham Pharmacia Biotech, Promega, and US Biochemical).

Suitable reporter molecules or labels which can be used for ease of detection include radionuclides, enzymes, and fluorescent, chemiluminescent, or chromogenic agents, as well as substrates, cofactors, inhibitors, magnetic particles, and the like.

*Expression and Purification of Polypeptides*

Host cells transformed with nucleotide sequences encoding a DA-like GPCR polypeptide can be cultured under conditions suitable for the expression and recovery of the protein from cell culture. The polypeptide produced by a transformed cell can be secreted or contained intracellularly depending on the sequence and/or the vector used. As will be understood by those of skill in the art, expression vectors containing polynucleotides which encode DA-like GPCR polypeptides can be designed to contain signal sequences which direct secretion of soluble DA-like GPCR polypeptides through a prokaryotic or eukaryotic cell membrane or which direct the membrane insertion of membrane-bound DA-like GPCR polypeptide.

As discussed above, other constructions can be used to join a sequence encoding a DA-like GPCR polypeptide to a nucleotide sequence encoding a polypeptide domain which will facilitate purification of soluble proteins. Such purification facilitating domains include, but are not limited to, metal chelating peptides such as histidine-tryptophan modules that allow purification on immobilized metals, protein A domains that allow purification on immobilized immunoglobulin, and the domain utilized in the FLAGS extension/affinity purification system (Immunex Corp., Seattle, Wash.). Inclusion of cleavable linker sequences such as those specific for Factor Xa or enterokinase (Invitrogen, San Diego, CA) between the purification domain and the DA-like GPCR polypeptide also can be used to facilitate purification. One such expression vector provides for expression of a fusion protein containing a DA-like GPCR polypeptide and 6 histidine residues preceding a thioredoxin or an enterokinase cleavage site. The histidine residues facilitate purification by IMAC (immobilized metal ion affinity chromatography, as described in Porath *et al., Prot. Exp. Purif. 3*, 263-281, 1992), while the enterokinase cleavage site provides a means for purifying the DA-like GPCR polypeptide from the fusion protein. Vectors which contain fusion proteins are disclosed in Kroll *et al., DNA Cell Biol. 12*, 441-453, 1993.

*Chemical Synthesis*

Sequences encoding a DA-like GPCR polypeptide can be synthesized, in whole or in part, using chemical methods well known in the art (see Caruthers *et al., Nucl. Acids Res. Symp. Ser.* 215-223, 1980; Hom *et al. Nucl. Acids Res. Symp. Ser.* 225-232, 1980). Alternatively, a DA-like GPCR polypeptide itself can be produced using chemical methods to synthesize its amino acid sequence, such as by direct peptide synthesis using solid-phase techniques (Merrifield, *J. Am. Chem. Soc. 85*, 2149-2154, 1963; Roberge *et al., Science 269,* 202-204, 1995). Protein synthesis can be performed using manual techniques or by automation. Automated synthesis can be achieved, for example, using Applied Biosystems 431A Peptide Synthesizer (Perkin Elmer). Optionally, fragments of DA-like GPCR polypeptides can be separately synthesized and combined using chemical methods to produce a full-length molecule.

The newly synthesized peptide can be substantially purified by preparative high performance liquid chromatography (*e.g.*, Creighton, PROTEINS: STRUCTURES AND MOLECULAR PRINCIPLES, WH Freeman and Co., New York, N.Y., 1983). The composition of a synthetic DA-like GPCR polypeptide can be confirmed by amino acid analysis or sequencing (*e.g.*, the Edman degradation procedure; *see* Creighton, *supra*). Additionally, any portion of the amino acid sequence of the DA-like GPCR polypeptide can be altered during direct synthesis and/or combined using chemical methods with sequences from other proteins to produce a variant polypeptide or a fusion protein.

*Production of Altered Polypeptides*

As will be understood by those of skill in the art, it may be advantageous to produce DA-like GPCR polypeptide-encoding nucleotide sequences possessing non-naturally occurring codons. For example, codons preferred by a particular prokaryotic or eukaryotic host can be selected to increase the rate of protein expression or to produce an RNA transcript having desirable properties, such as a half-life which is longer than that of a transcript generated from the naturally occurring sequence.

The nucleotide sequences disclosed herein can be engineered using methods generally known in the art to alter DA-like GPCR polypeptide-encoding sequences for a variety of reasons, including but not limited to, alterations which modify the cloning, processing, and/or expression of the polypeptide or mRNA product. DNA shuffling by random fragmentation and PCR reassembly of gene fragments and synthetic oligonucleotides can be used to engineer the nucleotide sequences. For example, site-directed mutagenesis can be used to insert new restriction sites, alter glycosylation patterns, change codon preference, produce splice variants, introduce mutations, and so forth.

*Antibodies*

Any type of antibody known in the art can be generated to bind specifically to an epitope of a DA-like GPCR polypeptide. "Antibody" as used herein includes intact immunoglobulin molecules, as well as fragments thereof, such as Fab, $F(ab')_2$, and Fv, which are capable of binding an epitope of a DA-like GPCR polypeptide. Typically, at least 6, 8, 10, or 12 contiguous amino acids are required to form an epitope. However, epitopes which involve non-contiguous amino acids may require more, e.g., at least 15, 25, or 50 amino acids.

An antibody which specifically binds to an epitope of a DA-like GPCR polypeptide can be used therapeutically, as well as in immunochemical assays, such as Western blots, ELISAs, radioimmunoassays, immunohistochemical assays, immuno-precipitations, or other immunochemical assays known in the art. Various immunoassays can be used to identify antibodies having the desired specificity. Numerous protocols for competitive binding or immunoradiometric assays are well known in the art. Such immunoassays typically involve the measurement of complex formation between an immunogen and an antibody which specifically binds to the immunogen.

Typically, an antibody which specifically binds to a DA-like GPCR polypeptide provides a detection signal at least 5-, 10-, or 20-fold higher than a detection signal provided with other proteins when used in an immunochemical assay. Preferably, antibodies which specifically bind to DA-like GPCR polypeptides do not detect other proteins in immunochemical assays and can immunoprecipitate a DA-like GPCR polypeptide from solution.

DA-like GPCR polypeptides can be used to immunize a mammal, such as a mouse, rat, rabbit, guinea pig, monkey, or human, to produce polyclonal antibodies. If desired, a DA-like GPCR polypeptide can be conjugated to a carrier protein, such as bovine serum albumin, thyroglobulin, and keyhole limpet hemocyanin. Depending on the host species, various adjuvants can be used to increase the immunological response. Such adjuvants include, but are not limited to, Freund's adjuvant, mineral gels (*e.g.*, aluminum hydroxide), and surface active substances (*e.g.* lysolecithin, pluronic polyols, polyanions, peptides, oil emulsions, keyhole limpet hemocyanin, and dinitrophenol). Among adjuvants used in humans, BCG (*bacilli Calmette-Guerin*) and *Corynebacterium parvum* are especially useful.

Monoclonal antibodies which specifically bind to a DA-like GPCR polypeptide can be prepared using any technique which provides for the production of antibody molecules by continuous cell lines in culture. These techniques include, but are not limited to, the hybridoma technique, the human B-cell hybridoma technique, and the EBV-hybridoma technique (Kohler *et al., Nature 256*, 495-497, 1985; Kozbor *et al., J. Immunol. Methods 81,* 31-42, 1985; Cote *et al., Proc. Natl. Acad. Sci. 80,* 2026-2030, 1983; Cole *et al., Mol. Cell Biol. 62*, 109-120, 1984).

In addition, techniques developed for the production of "chimeric antibodies," the splicing of mouse antibody genes to human antibody genes to obtain a molecule with appropriate antigen specificity and biological activity, can be used (Morrison *et al., Proc. Natl. Acad. Sci. 81,* 6851-6855, 1984; Neuberger *et al., Nature 312,* 604-608, 1984; Takeda *et al., Nature 314,* 452-454, 1985). Monoclonal and other antibodies also can be "humanized" to prevent a patient from mounting an immune response against the antibody when it is used therapeutically. Such antibodies may be sufficiently similar in sequence to human antibodies to be used directly in therapy or may require alteration of a few key residues. Sequence differences between rodent antibodies and human sequences can be minimized by replacing residues which differ from those in the human sequences by site directed mutagenesis of individual residues or by grating of entire complementarity determining regions. Alternatively, humanized antibodies can be produced using recombinant methods, as described in GB2188638B. Antibodies which specifically bind to a DA-like GPCR polypeptide can contain antigen binding sites which are either partially or fully humanized, as disclosed in U.S. 5,565,332.

Alternatively, techniques described for the production of single chain antibodies can be adapted using methods known in the art to produce single chain antibodies which specifically bind to DA-like GPCR polypeptides. Antibodies with related specificity, but of distinct idiotypic composition, can be generated by chain shuffling from random combinatorial immunoglobin libraries (Burton, *Proc. Natl. Acad. Sci. 88,* 11120-23, 1991).

Single-chain antibodies also can be constructed using a DNA amplification method, such as PCR, using hybridoma cDNA as a template (Thirion *et al.,* 1996, *Eur. J. Cancer Prev. 5*, 507-11). Single-chain antibodies can be mono- or bispecific, and can be bivalent or tetravalent. Construction of tetravalent, bispecific single-chain antibodies is taught, for example, in Coloma & Morrison, 1997, *Nat. Biotechnol. 15,* 159-63. Construction of bivalent, bispecific single-chain antibodies is taught in Mallender & Voss, 1994, *J. Biol. Chem. 269*, 199-206.

A nucleotide sequence encoding a single-chain antibody can be constructed using manual or automated nucleotide synthesis, cloned into an expression construct using standard recombinant DNA methods, and introduced into a cell to express the coding sequence, as described below. Alternatively, single-chain antibodies can be produced directly using, for example, filamentous phage technology (*Verhaar et al.,* 1995, *Int. J. Cancer 61,* 497-501; Nicholls *et al.,* 1993, *J. Immunol. Meth. 165,* 81-91).

Antibodies which specifically bind to DA-like GPCR polypeptides also can be produced by inducing *in vivo* production in the lymphocyte population or by screening immunoglobulin libraries or panels of highly specific binding reagents as disclosed in the literature (Orlandi *et al., Proc. Natl. Acad Sci. 86*, 3833-3837, 1989; *Winter et al., Nature 349,* 293-299, 1991).

Other types of antibodies can be constructed and used therapeutically in methods of the invention. For example, chimeric antibodies can be constructed as disclosed in WO 93/03151. Binding proteins which are derived from immunoglobulins and which are multivalent and multispecific, such as the "diabodies" described in WO 94/13804, also can be prepared.

Antibodies according to the invention can be purified by methods well known in the art. For example, antibodies can be affinity purified by passage over a column to which a DA-like GPCR polypeptide is bound. The bound antibodies can then be eluted from the column using a buffer with a high salt concentration.

## *Antisense Oligonucleotides*

Antisense oligonucleotides are nucleotide sequences which are complementary to a specific DNA or RNA sequence. Once introduced into a cell, the complementary nucleotides combine with natural sequences produced by the cell to form complexes and block either transcription or translation. Preferably, an antisense oligonucleotide is at least 11 nucleotides in length, but can be at least 12, 15, 20, 25, 30, 35, 40, 45, or 50 or more nucleotides long. Longer sequences also can be used. Antisense oligonucleotide molecules can be provided in a DNA construct and introduced into a cell as described above to decrease the level of DA-like GPCR gene products in the cell.

Antisense oligonucleotides can be deoxyribonucleotides, ribonucleotides, or a combination of both. Oligonucleotides can be synthesized manually or by an automated synthesizer, by covalently linking the 5' end of one nucleotide with the 3' end of another nucleotide with non-phosphodiester internucleotide linkages such alkylphosphonates, phosphorothioates, phosphorodithioates, alkylphosphonothioates, alkylphosphonates, phosphoramidates, phosphate esters, carbamates, acetamidate, carboxymethyl esters, carbonates, and phosphate triesters. *See* Brown, *Meth. Mol. Biol. 20,* 1-8, 1994; Sonveaux, *Meth. Mol. Biol. 26,* 1-72, 1994; Uhlmann *et al., Chem. Rev. 90,* 543-583, 1990.

Modifications of DA-like GPCR gene expression can be obtained by designing antisense oligonucleotides which will form duplexes to the control, 5', or regulatory regions of the DA-like GPCR gene. Oligonucleotides derived from the transcription initiation site, e.g., between positions -10 and +10 from the start site, are preferred. Similarly, inhibition can be achieved using "triple helix" base-pairing methodology. Triple helix pairing is useful because it causes inhibition of the ability of the double helix to open sufficiently for the binding of polymerases, transcription factors, or chaperons. Therapeutic advances using triplex DNA have been described in the literature (*e.g.,* Gee *et al.,* in Huber & Carr, MOLECULAR AND IMMUNOLOGIC APPROACHES, Futura Publishing Co., Mt. Kisco, N.Y., 1994). An antisense oligonucleotide also can be designed to block translation of mRNA by preventing the transcript from binding to ribosomes.

Precise complementarity is not required for successful complex formation between an antisense oligonucleotide and the complementary sequence of a DA-like GPCR polynucleotide. Antisense oligonucleotides which comprise, for example, 2, 3, 4, or 5 or more stretches of contiguous nucleotides which are precisely complementary to a DA-like GPCR polynucleotide, each separated by a stretch of contiguous nucleotides which are not complementary to adjacent DA-like GPCR nucleotides, can provide sufficient targeting specificity for DA-like GPCR mRNA. Preferably, each stretch of complementary contiguous nucleotides is at least 4, 5, 6, 7, or 8 or more nucleotides in length. Non-complementary intervening sequences are preferably 1, 2, 3, or 4 nucleotides in length. One skilled in the art can easily use the calculated melting point of an antisense-sense pair to determine the degree of mismatching which will be tolerated between a particular antisense oligonucleotide and a particular DA-like GPCR polynucleotide sequence.

Antisense oligonucleotides can be modified without affecting their ability to hybridize to a DA-like GPCR polynucleotide. These modifications can be internal or at one or both ends of the antisense molecule. For example, internucleoside phosphate linkages can be modified by adding cholesteryl or diamine moieties with varying numbers of carbon residues between the amino groups and terminal ribose. Modified bases and/or sugars, such as arabinose instead of ribose, or a 3', 5'-substituted oligonucleotide in which the 3' hydroxyl group or the 5' phosphate group are substituted, also can be employed in a modified antisense oligonucleotide. These modified oligonucleotides can be prepared by methods well known in the art. *See, e.g.,* Agrawal *et al., Trends Biotechnol. 10,* 152-158, 1992; Uhlmann *et al., Chem. Rev. 90,* 543-584, 1990; Uhlmann *et al., Tetrahedron. Lett. 215,* 3539-3542, 1987.

*Ribozymes*

Ribozymes are RNA molecules with catalytic activity. *See, e.g.,* Cech, *Science 236,* 1532-1539; 1987; Cech, *Ann. Rev. Biochem. 59,* 543-568; 1990, Cech, *Curr. Opin. Struct. Biol. 2*, 605-609; 1992, Couture & Stinchcomb, *Trends Genet. 12,* 510-515, 1996. Ribozymes can be used to inhibit gene function by cleaving an RNA sequence, as is known in the art (*e.g.*, Haseloff *et al.,* U.S. Patent 5,641,673). The mechanism of ribozyme action involves sequence-specific hybridization of the ribozyme molecule to complementary target RNA, followed by endonucleolytic cleavage. Examples include engineered hammerhead motif ribozyme molecules that can specifically and efficiently catalyze endonucleolytic cleavage of specific nucleotide sequences.

The coding sequence of a DA-like GPCR polynucleotide, such as the nucleotide sequence shown in SEQ ID NO. 1, can be used to generate ribozymes which will specifically bind to mRNA transcribed from the DA-like GPCR polynucleotide. Methods of designing and constructing ribozymes which can cleave other RNA molecules in trans in a highly sequence specific manner have been developed and described in the art (*see* Haseloff *et al. Nature 334,* 585-591, 1988). For example, the cleavage activity of ribozymes can be targeted to specific RNAs by engineering a discrete "hybridization" region into the ribozyme. The hybridization region contains a sequence complementary to the target RNA and thus specifically hybridizes with the target (see, for example, *Gerlach et al*., EP 321,201).

Specific ribozyme cleavage sites within a DA-like GPCR RNA target can be identified by scanning the target molecule for ribozyme cleavage sites which include the following sequences: GUA, GUU, and GUC. Once identified, short RNA sequences of between 15 and 20 ribonucleotides corresponding to the region of the target RNA containing the cleavage site can be evaluated for secondary structural features which may render the target inoperable. Suitability of candidate DA-like GPCR RNA targets also can be evaluated by testing accessibility to hybridization with complementary oligonucleotides using ribonuclease protection assays. The nucleotide sequence shown in SEQ ID NO. 1 and its complement provide a source of suitable hybridization region sequences. Longer complementary sequences can be used to increase the affinity of the hybridization sequence for the target. The hybridizing and cleavage regions of the ribozyme can be integrally related such that upon hybridizing to the target RNA through the complementary regions, the catalytic region of the ribozyme can cleave the target.

Ribozymes can be introduced into cells as part of a DNA construct. Mechanical methods, such as microinjection, liposome-mediated transfection, electroporation, or calcium phosphate precipitation, can be used to introduce a ribozyme-containing DNA construct into cells in which it is desired to decrease DA-like GPCR expression. Alternatively, if it is desired that the cells stably retain the DNA construct, the construct can be supplied on a plasmid and maintained as a separate element or integrated into the genome of the cells, as is known in the art. A ribozyme-encoding DNA construct can include transcriptional regulatory elements, such as a promoter element, an enhancer or UAS element, and a transcriptional terminator signal, for controlling transcription of ribozymes in the cells.

As taught in Haseloff *et al.,* U.S. Patent 5,641,673, ribozymes can be engineered so that ribozyme expression will occur in response to factors which induce expression of a target gene. Ribozymes also can be engineered to provide an additional level of regulation, so that destruction of mRNA occurs only when both a ribozyme and a target gene are induced in the cells.

*Screening Methods*

The invention provides assays for screening test compounds which bind to or modulate the activity of a DA-like GPCR polypeptide or a DA-like GPCR polynucleotide. A test compound preferably binds to a DA-like GPCR polypeptide or polynucleotide. More preferably, a test compound decreases or increases the effect of dopamine or a dopamine analog as mediated via human DA-like GPCR by at least about 10, preferably about 50, more preferably about 75, 90, or 100% relative to the absence of the test compound.

*Test Compounds*

Test compounds can be pharmacologic agents already known in the art or can be compounds previously unknown to have any pharmacological activity. The compounds can be naturally occurring or designed in the laboratory. They can be isolated from microorganisms, animals, or plants, and can be produced recombinantly, or synthesized by chemical methods known in the art. If desired, test compounds can be obtained using any of the numerous combinatorial library methods known in the art, including but not limited to, biological libraries, spatially addressable parallel solid phase or solution phase libraries, synthetic library methods requiring deconvolution, the "one-bead one-compound" library method, and synthetic library methods using affinity chromatography selection. The biological library approach is limited to polypeptide libraries, while the other four approaches are applicable to polypeptide, non-peptide oligomer, or small molecule libraries of compounds. *See* Lam, *Anticancer Drug Des. 12*, 145, 1997.

Methods for the synthesis of molecular libraries are well known in the art (*see,* for example, DeWitt *et al., Proc. Natl. Acad. Sci. U.S.A. 90,* 6909, 1993; Erb *et al. Proc. Natl. Acad. Sci. U.S.A. 91,* 11422, 1994; Zuckermann *et al., J Med. Chem. 37*, 2678, 1994; Cho *et al., Science 261,* 1303, 1993; Carell *et al., Angew. Chem. Int. Ed. Engl. 33*, 2059, 1994; Carell *et al., Angew. Chem. Int. Ed. Engl. 33,* 2061; Gallop *et al., J. Med. Chem. 37,* 1233, 1994). Libraries of compounds can be presented in solution (*see, e.g.,* Houghten, *BioTechniques 13,* 412-421, 1992), or on beads (Lam, *Nature 354,* 82-84, 1991), chips (Fodor, *Nature 364,* 555-556, 1993), bacteria or spores (Ladner, U.S. Patent 5,223,409), plasmids (Cull *et al., Proc. Natl. Acad. Sci. U.S.A. 89,* 1865-1869, 1992), or phage (Scott & Smith, *Science 249,* 386-390, 1990; Devlin, *Science 249,* 404-406, 1990); Cwirla *et al., Proc. Natl. Acad. Sci. 97,* 6378-6382, 1990; Felici, *J. Mol. Biol. 222*, 301-310, 1991; and Ladner, U.S. Patent 5,223,409).

*High Throughput Screening*

Test compounds can be screened for the ability to bind to DA-like GPCR polypeptides or polynucleotides or to affect DA-like GPCR activity or DA-like GPCR gene expression using high throughput screening. Using high throughput screening, many discrete compounds can be tested in parallel so that large numbers of test compounds can be quickly screened. The most widely established techniques utilize 96-well microtiter plates. The wells of the microtiter plates typically require assay volumes that range from 50 to 500 μl. In addition to the plates, many instruments, materials, pipettors, robotics, plate washers, and plate readers are commercially available to fit the 96-well format.

Alternatively, "free format assays," or assays that have no physical barrier between samples, can be used. For example, an assay using pigment cells (melanocytes) in a simple homogeneous assay for combinatorial peptide libraries is described by Jayawickreme *et al., Proc. Natl. Acad. Sci. U.S.A. 19,* 1614-18 (1994). The cells are placed under agarose in petri dishes, then beads that carry combinatorial compounds are placed on the surface of the agarose. The combinatorial compounds are partially released the compounds from the beads. Active compounds can be visualized as dark pigment areas because, as the compounds diffuse locally into the gel matrix, the active compounds cause the cells to change colors.

Another example of a free format assay is described by Chelsky, "Strategies for Screening Combinatorial Libraries: Novel and Traditional Approaches," reported at the First Annual Conference of The Society for Biomolecular Screening in Philadelphia, Pa. (Nov. 7-10, 1995). Chelsky placed a simple homogenous enzyme assay for carbonic anhydrase inside an agarose gel such that the enzyme in the gel would cause a color change throughout the gel. Thereafter, beads carrying combinatorial compounds via a photolinker were placed inside the gel and the compounds were partially released by UV-light. Compounds that inhibited the enzyme were observed as local zones of inhibition having less color change.

Yet another example is described by Salmon *et al*., *Molecular Diversity 2*, 57-63 (1996). In this example, combinatorial libraries were screened for compounds that had cytotoxic effects on cancer cells growing in agar.

Another high throughput screening method is described in Beutel *et al.,* U.S. Patent 5,976,813. In this method, test samples are placed in a porous matrix. One or more assay components are then placed within, on top of, or at the bottom of a matrix such as a gel, a plastic sheet, a filter, or other form of easily manipulated solid support. When samples are introduced to the porous matrix they diffuse sufficiently slowly, such that the assays can be performed without the test samples running together.

*Binding Assays*

For binding assays, the test compound is preferably a small molecule which binds to and occupies the active site of the DA-like GPCR polypeptide, thereby making the ligand binding site inaccessible to substrate such that normal biological activity is prevented. Examples of such small molecules include, but are not limited to, small peptides or peptide-like molecules. Potential ligands which bind to a polypeptide of the invention include, but are not limited to, the natural ligands of known DA-like GPCRs and analogues or derivatives thereof.

In binding assays, either the test compound or the DA-like GPCR polypeptide can comprise a detectable label, such as a fluorescent, radioisotopic, chemiluminescent, or enzymatic label, such as horseradish peroxidase, alkaline phosphatase, or luciferase. Detection of a test compound which is bound to the DA-like GPCR polypeptide can then be accomplished, for example, by direct counting of radioemmission, by scintillation counting, or by determining conversion of an appropriate substrate to a detectable product.

Alternatively, binding of a test compound to a DA-like GPCR polypeptide can be determined without labeling either of the interactants. For example, a microphysiometer can be used to detect binding of a test compound with a DA-like GPCR polypeptide. A microphysiometer (*e.g.,* Cytosensor™) is an analytical instrument that measures the rate at which a cell acidifies its environment using a light-addressable potentiometric sensor (LAPS). Changes in this acidification rate can be used as an indicator of the interaction between a test compound and a DA-like GPCR polypeptide (McConnell *et al., Science 257*, 1906-1912, 1992).

Determining the ability of a test compound to bind to a DA-like GPCR polypeptide also can be accomplished using a technology such as real-time Bimolecular Interaction Analysis (BIA) (Sjolander & Urbaniczky, *Anal. Chem. 63,* 2338-2345, 1991, and Szabo *et al., Curr. Opin. Struct. Biol. 5*, 699-705, 1995). BIA is a technology for studying biospecific interactions in real time, without labeling any of the interactants (*e.g.*, BIAcore™). Changes in the optical phenomenon surface plasmon resonance (SPR) can be used as an indication of real-time reactions between biological molecules.

In yet another aspect of the invention, a DA-like GPCR polypeptide can be used as a "bait protein" in a two-hybrid assay or three-hybrid assay (see, *e.g.*, U.S. Patent 5,283,317; Zervos *et al., Cell 72,* 223-232, 1993; Madura *et al., J Biol. Chem. 268,* 12046-12054, 1993; Bartel *et al., BioTechniques 14,* 920-924, 1993; Iwabuchi *et al., Oncogene 8,* 1693-1696, 1993; and Brent W094/10300), to identify other proteins which bind to or interact with the DA-GPCR polypeptide and modulate its activity.

The two-hybrid system is based on the modular nature of most transcription factors, which consist of separable DNA-binding and activation domains. Briefly, the assay utilizes two different DNA constructs. For example, in one construct, polynucleotide encoding a DA-like GPCR polypeptide can be fused to a polynucleotide encoding the DNA binding domain of a known transcription factor (*e.g.*, GAL-4). In the other construct a DNA sequence that encodes an unidentified protein ("prey" or "sample") can be fused to a polynucleotide that codes for the activation domain of the known transcription factor. If the "bait" and the "prey" proteins are able to interact *in vivo* to form an protein-dependent complex, the DNA-binding and activation domains of the transcription factor are brought into close proximity. This proximity allows transcription of a reporter gene (*e.g.*, LacZ), which is operably linked to a transcriptional regulatory site responsive to the transcription factor. Expression of the reporter gene can be detected, and cell colonies containing the functional transcription factor can be isolated and used to obtain the DNA sequence encoding the protein which interacts with the DA-like GPCR polypeptide.

It may be desirable to immobilize either the DA-like GPCR polypeptide (or polynucleotide) or the test compound to facilitate separation of bound from unbound forms of one or both of the interactants, as well as to accommodate automation of the assay. Thus, either the DA-like GPCR polypeptide (or polynucleotide) or the test compound can be bound to a solid support. Suitable solid supports include, but are not limited to, glass or plastic slides, tissue culture plates, microtiter wells, tubes, silicon chips, or particles such as beads (including, but not limited to, latex, polystyrene, or glass beads). Any method known in the art can be used to attach the DA-like GPCR polypeptide (or polynucleotide) or test compound to a solid support, including use of covalent and non-covalent linkages, passive absorption, or pairs of binding moieties attached respectively to the polypeptide (or polynucleotide) or test compound and the solid support. Test compounds are preferably bound to the solid support in an array, so that the location of individual test compounds can be tracked. Binding of a test compound to a DA-like GPCR polypeptide (or polynucleotide) can be accomplished in any vessel suitable for containing the reactants. Examples of such vessels include microtiter plates, test tubes, and microcentrifuge tubes.

In one embodiment, the DA-like GPCR polypeptide is a fusion protein comprising a domain that allows the DA-like GPCR polypeptide to be bound to a solid support. For example, glutathione-S-transferase fusion proteins can be adsorbed onto glutathione sepharose beads (Sigma Chemical, St. Louis, Mo.) or glutathione derivatized microtiter plates, which are then combined with the test compound or the test compound and the non-adsorbed DA-like GPCR polypeptide; the mixture is then incubated under conditions conducive to complex formation (*e.g.*, at physiological conditions for salt and pH). Following incubation, the beads or microtiter plate wells are washed to remove any unbound components. Binding of the interactants can be determined either directly or indirectly, as described above. Alternatively, the complexes can be dissociated from the solid support before binding is determined.

Other techniques for immobilizing proteins or polynucleotides on a solid support also can be used in the screening assays of the invention. For example, either a DA-like GPCR polypeptide (or polynucleotide) or a test compound can be immobilized utilizing conjugation of biotin and streptavidin. Biotinylated DA-like GPCR polypeptides (or polynucleotides) or test compounds can be prepared from biotin-NHS(N-hydroxysuccinimide) using techniques well known in the art (*e.g.*, biotinylation kit, Pierce Chemicals, Rockford, Ill.) and immobilized in the wells of streptavidin-coated 96 well plates (Pierce Chemical). Alternatively, antibodies which specifically bind to a DA-like GPCR polypeptide, polynucleotide, or a test compound, but which do not interfere with a desired binding site, such as the active site of the DA-like GPCR polypeptide, can be derivatized to the wells of the plate. Unbound target or protein can be trapped in the wells by antibody conjugation.

Methods for detecting such complexes, in addition to those described above for the GST-immobilized complexes, include immunodetection of complexes using antibodies which specifically bind to the DA-like GPCR polypeptide or test compound, enzyme-linked assays which rely on detecting an activity of the DA-like GPCR polypeptide, and SDS gel electrophoresis under non-reducing conditions.

Screening for test compounds which bind to a DA-like GPCR polypeptide or polynucleotide also can be carried out in an intact cell. Any cell which comprises a DA-like GPCR polypeptide or polynucleotide can be used in a cell-based assay system. A DA-like GPCR polynucleotide can be naturally occurring in the cell or can be introduced using techniques such as those described above. Binding of the test compound to a DA-like GPCR polypeptide or polynucleotide is determined as described above.

*Functional Assays*

Test compounds can be tested for the ability to increase or decrease a biological effect of a DA-like GPCR polypeptide. Such biological effects can be determined using the functional assays described in the specific examples, below. Functional assays can be carried out after contacting either a purified DA-like GPCR polypeptide, a cell membrane preparation, or an intact cell with a test compound. A test compound which decreases a functional activity of a DA-like GPCR by at least about 10, preferably about 50, more preferably about 75, 90, or 100% is identified as a potential agent for decreasing DA-like GPCR activity. A test compound which increases DA-like GPCR activity by at least about 10, preferably about 50, more preferably about 75, 90, or 100% is identified as a potential agent for increasing DA-GPCR activity.

One such screening procedure involves the use of melanophores which are transfected to express a DA-GPCR polypeptide. Such a screening technique is described in WO 92/01810 published Feb. 6, 1992. Thus, for example, such an assay may be employed for screening for a compound which inhibits activation of the receptor polypeptide by contacting the melanophore cells which comprise the receptor with both the receptor ligand (*e.g.*, dopamine or a dopamine analog) and a test compound to be screened. Inhibition of the signal generated by the ligand indicates that a test compound is a potential antagonist for the receptor, *i.e.,* inhibits activation of the receptor. The screen may be employed for identifying a test compound which activates the receptor by contacting such cells with compounds to be screened and determining whether each test compound generates a signal, i. e. , activates the receptor.

Other screening techniques include the use of cells which express a human DA-like GPCR polypeptide (for example, transfected CHO cells) in a system which measures extracellular pH changes caused by receptor activation (*see, e.g., Science 246,* 181-296, 1989). For example, test compounds may be contacted with a cell which expresses a human DA-like GPCR polypeptide and a second messenger response, e.g., signal transduction or pH changes, can be measured to determine whether the test compound activates or inhibits the receptor.

Another such screening technique involves introducing RNA encoding a human DA-like GPCR polypeptide into *Xenopus* oocytes to transiently express the receptor. The transfected oocytes can then be contacted with the receptor ligand and a test compound to be screened, followed by detection of inhibition or activation of a calcium signal in the case of screening for test compounds which are thought to inhibit activation of the receptor.

Another screening technique involves expressing a human DA-like GPCR polypeptide in cells in which the receptor is linked to a phospholipase C or D. Such cells include endothelial cells, smooth muscle cells, embryonic kidney cells, etc. The screening may be accomplished as described above by quantifying the degree of activation of the receptor from changes in the phospholipase activity.

Details of functional assays such as those described above are provided in the specific examples, below.

*Gene Expression*

In another embodiment, test compounds which increase or decrease DA-like GPCR gene expression are identified. A DA-like GPCR polynucleotide is contacted with a test compound, and the expression of an RNA or polypeptide product of the DA-like GPCR polynucleotide is determined. The level of expression of appropriate mRNA or polypeptide in the presence of the test compound is compared to the level of expression of mRNA or polypeptide in the absence of the test compound. The test compound can then be identified as a modulator of expression based on this comparison. For example, when expression of mRNA or polypeptide is greater in the presence of the test compound than in its absence, the test compound is identified as a stimulator or enhancer of the mRNA or polypeptide expression. Alternatively, when expression of the mRNA or polypeptide is less in the presence of the test compound than in its absence, the test compound is identified as an inhibitor of the mRNA or polypeptide expression.

The level of DA-like GPCR mRNA or polypeptide expression in the cells can be determined by methods well known in the art for detecting mRNA or polypeptide. Either qualitative or quantitative methods can be used. The presence of polypeptide products of a DA-like GPCR polynucleotide can be determined, for example, using a variety of techniques known in the art, including immunochemical methods such as radioimmunoassay, Western blotting, and immunohistochemistry. Alternatively, polypeptide synthesis can be determined *in vivo,* in a cell culture, or in an *in vitro* translation system by detecting incorporation of labeled amino acids into a DA-like GPCR polypeptide.

Such screening can be carried out either in a cell-free assay system or in an intact cell. Any cell which expresses a DA-like GPCR polynucleotide can be used in a cell-based assay system. The DA-like GPCR polynucleotide can be naturally occurring in the cell or can be introduced using techniques such as those described above. Either a primary culture or an established cell line, such as CHO or human embryonic kidney 293 cells, can be used.

*Pharmaceutical Compositions*

The invention also provides pharmaceutical compositions which can be administered to a patient to achieve a therapeutic effect. Pharmaceutical compositions of the invention can comprise, for example, a DA-like GPCR polypeptide, DA-like GPCR polynucleotide, antibodies which specifically bind to a DA-like GPCR polypeptide, or mimetics, agonists, antagonists, or inhibitors of a DA-like GPCR polypeptide activity. The compositions can be administered alone or in combination with at least one other agent, such as stabilizing compound, which can be administered in any sterile, biocompatible pharmaceutical carrier, including, but not limited to, saline, buffered saline, dextrose, and water. The compositions can be administered to a patient alone, or in combination with other agents, drugs or hormones.

In addition to the active ingredients, these pharmaceutical compositions can contain suitable pharmaceutically-acceptable carriers comprising excipients and auxiliaries which facilitate processing of the active compounds into preparations which can be used pharmaceutically. Pharmaceutical compositions of the invention can be administered by any number of routes including, but not limited to, oral, intravenous, intramuscular, intra-arterial, intramedullary, intrathecal, intraventricular, transdermal, subcutaneous, intraperitoneal, intranasal, parenteral, topical, sublingual, or rectal means. Pharmaceutical compositions for oral administration can be formulated using pharmaceutically acceptable carriers well known in the art in dosages suitable for oral administration. Such carriers enable the pharmaceutical compositions to be formulated as tablets, pills, dragees, capsules, liquids, gels, syrups, slurries, suspensions, and the like, for ingestion by the patient.

Pharmaceutical preparations for oral use can be obtained through combination of active compounds with solid excipient, optionally grinding a resulting mixture, and processing the mixture of granules, after adding suitable auxiliaries, if desired, to obtain tablets or dragee cores. Suitable excipients are carbohydrate or protein fillers, such as sugars, including lactose, sucrose, mannitol, or sorbitol; starch from corn, wheat, rice, potato, or other plants; cellulose, such as methyl cellulose, hydroxypropylmethyl-cellulose, or sodium carboxymethylcellulose; gums including arabic and tragacanth; and proteins such as gelatin and collagen. If desired, disintegrating or solubilizing agents can be added, such as the cross-linked polyvinyl pyrrolidone, agar, alginic acid, or a salt thereof, such as sodium alginate.

Dragee cores can be used in conjunction with suitable coatings, such as concentrated sugar solutions, which also can contain gum arabic, talc, polyvinylpyrrolidone, carbopol gel, polyethylene glycol, and/or titanium dioxide, lacquer solutions, and suitable organic solvents or solvent mixtures. Dyestuffs or pigments can be added to the tablets or dragee coatings for product identification or to characterize the quantity of active compound, *i.e.,* dosage.

Pharmaceutical preparations which can be used orally include push-fit capsules made of gelatin, as well as soft, sealed capsules made of gelatin and a coating, such as glycerol or sorbitol. Push-fit capsules can contain active ingredients mixed with a filler or binders, such as lactose or starches, lubricants, such as talc or magnesium stearate, and, optionally, stabilizers. In soft capsules, the active compounds can be dissolved or suspended in suitable liquids, such as fatty oils, liquid, or liquid polyethylene glycol with or without stabilizers.

Pharmaceutical formulations suitable for parenteral administration can be formulated in aqueous solutions, preferably in physiologically compatible buffers such as Hanks' solution, Ringer's solution, or physiologically buffered saline. Aqueous injection suspensions can contain substances which increase the viscosity of the suspension, such as sodium carboxymethyl cellulose, sorbitol, or dextran. Additionally, suspensions of the active compounds can be prepared as appropriate oily injection suspensions. Suitable lipophilic solvents or vehicles include fatty oils such as sesame oil, or synthetic fatty acid esters, such as ethyl oleate or triglycerides, or liposomes. Non-lipid polycationic amino polymers also can be used for delivery. Optionally, the suspension also can contain suitable stabilizers or agents which increase the solubility of the compounds to allow for the preparation of highly concentrated solutions. For topical or nasal administration, penetrants appropriate to the particular barrier to be permeated are used in the formulation. Such penetrants are generally known in the art.

The pharmaceutical compositions of the present invention can be manufactured in a manner that is known in the art, *e.g.*, by means of conventional mixing, dissolving, granulating, dragee-making, levigating, emulsifying, encapsulating, entrapping, or lyophilizing processes. The pharmaceutical composition can be provided as a salt and can be formed with many acids, including but not limited to, hydrochloric, sulfuric, acetic, lactic, tartaric, malic, succinic, etc.

Salts tend to be more soluble in aqueous or other protonic solvents than are the corresponding free base forms. In other cases, the preferred preparation can be a lyophilized powder which can contain any or all of the following: 1-50 mM histidine, 0.1%-2% sucrose, and 2-7% mannitol, at a pH range of 4.5 to 5.5, that is combined with buffer prior to use.

Further details on techniques for formulation and administration can be found in the latest edition of REMINGTON'S PHARMACEUTICAL SCIENCES (Maack Publishing Co., Easton, Pa.). After pharmaceutical compositions have been prepared, they can be placed in an appropriate container and labeled for treatment of an indicated condition. Such labeling would include amount, frequency, and method of administration.

*Therapeutic Indications and Methods*

GPCRs are ubiquitous in the mammalian host and are responsible for many biological functions, including many pathologies. Accordingly, it is desirable to find compounds and drugs which stimulate a GPCR on the one hand and which can inhibit the function of a GPCR on the other hand. For example, compounds which activate a GPCR may be employed for therapeutic purposes, such as the treatment of asthma, Parkinson's disease, acute heart failure, urinary retention, and osteoporosis. In particular, compounds which activate GPCRs are useful in treating various cardiovascular ailments such as caused by the lack of pulmonary blood flow or hypertension. In addition these compounds may also be used in treating various physiological disorders relating to abnormal control of fluid and electrolyte homeostasis and in diseases associated with abnormal angiotensin-induced aldosterone secretion.

In general, compounds which inhibit activation of a GPCR can be used for a variety of therapeutic purposes, for example, for the treatment of hypotension and/or hypertension angina pectoris, myocardial infarction, ulcers, asthma, allergies, benign prostatic hypertrophy, and psychotic and neurological disorders including schizophrenia, manic excitement, depression, delirium, dementia or severe mental retardation, dyskinesias, such as Huntington's disease or Tourett's syndrome, among others. Compounds which inhibit GPCRs also are useful in reversing endogenous anorexia, in the control of bulimia, and in treating various cardiovascular ailments such as caused by excessive pulmonary blood flow or hypotension. In particular, regulation of DA-like GPCR can be used to treat Alzheimer's disease.

This invention further pertains to the use of novel agents identified by the screening assays described above. Accordingly, it is within the scope of this invention to use a test compound identified as described herein in an appropriate animal model. For example, an agent identified as described herein (*e.g.*, a modulating agent, an antisense nucleic acid molecule, a specific antibody, ribozyme, or a DA-like GPCR polypeptide binding molecule) can be used in an animal model to determine the efficacy, toxicity, or side effects of treatment with such an agent. Alternatively, an agent identified as described herein can be used in an animal model to determine the mechanism of action of such an agent. Furthermore, this invention pertains to uses of novel agents identified by the above-described screening assays for treatments as described herein.

A reagent which affects DA-like GPCR activity can be administered to a human cell, either *in vitro* or *in vivo,* to reduce DA-like GPCR activity. The reagent preferably binds to an expression product of a human DA-like GPCR gene. If the expression product is a protein, the reagent is preferably an antibody. For treatment of human cells *ex vivo,* an antibody can be added to a preparation of stem cells except human embryonic stem cells which have been removed from the body. The cells can then be replaced in the same or another human body, with or without clonal propagation, as is known in the art.

In one embodiment, the reagent is delivered using a liposome. Preferably, the liposome is stable in the animal into which it has been administered for at least about 30 minutes, more preferably for at least about 1 hour, and even more preferably for at least about 24 hours. A liposome comprises a lipid composition that is capable of targeting a reagent, particularly a polynucleotide, to a particular site in an animal, such as a human. Preferably, the lipid composition of the liposome is capable of targeting to a specific organ of an animal, such as the lung, liver, spleen, heart brain, lymph nodes, and skin.

A liposome useful in the present invention comprises a lipid composition that is capable of fusing with the plasma membrane of the targeted cell to deliver its contents to the cell. Preferably, the transfection efficiency of a liposome is about 0.5 $\mu$g of DNA per 16 nmole of liposome delivered to about $10^6$ cells, more preferably about 1.0 $\mu$g of DNA per 16 nmole of liposome delivered to about $10^6$ cells, and even more preferably about 2.0 $\mu$g of DNA per 16 nmol of liposome delivered to about $10^6$ cells. Preferably, a liposome is between about 100 and 500 nm, more preferably between about 150 and 450 nm; and even more preferably between about 200 and 400 nm in diameter.

Suitable liposomes for use in the present invention include those liposomes standardly used in, for example, gene delivery methods known to those of skill in the art. More preferred liposomes include liposomes having a polycationic lipid composition and/or liposomes having a cholesterol backbone conjugated to polyethylene glycol. Optionally, a liposome comprises a compound capable of targeting the liposome to a tumor cell, such as a tumor cell ligand exposed on the outer surface of the liposome.

Complexing a liposome with a reagent such as an antisense oligonucleotide or ribozyme can be achieved using methods which are standard in the art (see, for example, U.S. Patent 5,705,151). Preferably, from about 0.1 $\mu$g to about

10 μg of polynucleotide is combined with about 8 nmol of liposomes, more preferably from about 0.5 μg to about 5 μg of polynucleotides are combined with about 8 nmol liposomes, and even more preferably about 1.0 μg of polynucleotides is combined with about 8 nmol liposomes.

In another embodiment, antibodies can be delivered to specific tissues *in vivo* using receptor-mediated targeted delivery. Receptor-mediated DNA delivery techniques are taught in, for example, Findeis *et al. Trends in Biotechnol. 11,* 202-05 (1993); Chiou *et al.,* GENE THERAPEUTICS: METHODS AND APPLICATIONS OF DIRECT GENE TRANSFER (J.A. Wolff, ed.) (1994); Wu & Wu, *J. Biol. Chem. 263,* 621-24 (1988); *Wu et al., J Biol. Chem. 269,* 542-46 (1994); Zenke *et al., Proc. Natl. Acad. Sci. U.S.A. 87*, 3655-59 (1990); Wu *et al., J. Biol. Chem. 266,* 338-42 (1991).

*Determination of a Therapeutically Effective Dose*

The determination of a therapeutically effective dose is well within the capability of those skilled in the art. A therapeutically effective dose refers to that amount of active ingredient which increases or decreases DA-like GPCR activity relative to the DA-like GPCR activity which occurs in the absence of the therapeutically effective dose.

For any compound, the therapeutically effective dose can be estimated initially either in cell culture assays or in animal models, usually mice, rabbits, dogs, or pigs. The animal model also can be used to determine the appropriate concentration range and route of administration. Such information can then be used to determine useful doses and routes for administration in humans.

Therapeutic efficacy and toxicity, *e.g.*, $ED_{50}$ (the dose therapeutically effective in 50% of the population) and $LD_{50}$ (the dose lethal to 50% of the population), can be determined by standard pharmaceutical procedures in cell cultures or experimental animals. The dose ratio of toxic to therapeutic effects is the therapeutic index, and it can be expressed as the ratio, $LD_{50}/ED_{50}$.

Pharmaceutical compositions which exhibit large therapeutic indices are preferred. The data obtained from cell culture assays and animal studies is used in formulating a range of dosage for human use. The dosage contained in such compositions is preferably within a range of circulating concentrations that include the $ED_{50}$ with little or no toxicity. The dosage varies within this range depending upon the dosage form employed, sensitivity of the patient, and the route of administration.

The exact dosage will be determined by the practitioner, in light of factors related to the subject that requires treatment. Dosage and administration are adjusted to provide sufficient levels of the active ingredient or to maintain the desired effect. Factors which can be taken into account include the severity of the disease state, general health of the subject, age, weight, and gender of the subject, diet, time and frequency of administration, drug combination(s), reaction sensitivities, and tolerance/response to therapy. Long-acting pharmaceutical compositions can be administered every 3 to 4 days, every week, or once every two weeks depending on the half-life and clearance rate of the particular formulation.

Normal dosage amounts can vary from 0.1 to 100,000 micrograms, up to a total dose of about 1 g, depending upon the route of administration. Guidance as to particular dosages and methods of delivery is provided in the literature and generally available to practitioners in the art. Those skilled in the art will employ different formulations for nucleotides than for proteins or their inhibitors. Similarly, delivery of polynucleotides or polypeptides will be specific to particular cells, conditions, locations, etc.

If the reagent is a single-chain antibody, polynucleotides encoding the antibody can be constructed and introduced into a cell either *ex vivo* or *in vivo* using well-established techniques including, but not limited to, transferrin-polycation-mediated DNA transfer, transfection with naked or encapsulated nucleic acids, liposome-mediated cellular fusion, intracellular transportation of DNA-coated latex beads, protoplast fusion, viral infection, electroporation, "gene gun," and DEAE- or calcium phosphate-mediated transfection.

Effective *in vivo* dosages of an antibody are in the range of about 5 μg to about 50 μg/kg, about 50 μg to about 5 mg/kg, about 100 μg to about 500 μg/kg of patient body weight, and about 200 to about 250μg/kg of patient body weight. For administration of polynucleotides encoding single-chain antibodies, effective *in vivo* dosages are in the range of about 100 ng to about 200 ng, 500 ng to about 50 mg, about 1 μg to about 2 mg, about 5 μg to about 500 μg, and about 20 μg to about 100 μg of DNA.

If the expression product is mRNA, the reagent is preferably an antisense oligonucleotide or a ribozyme. Polynucleotides which express antisense oligonucleotides or ribozymes can be introduced into cells by a variety of methods, as described above.

Preferably, a reagent reduces expression of a DA-like GPCR gene or the activity of a DA-GPCR polypeptide by at least about 10, preferably about 50, more preferably about 75, 90, or 100% relative to the absence of the reagent. The effectiveness of the mechanism chosen to decrease the level of expression of a DA-like GPCR gene or the activity of a DA-like GPCR polypeptide can be assessed using methods well known in the art, such as hybridization of nucleotide probes to DA-like GPCR-specific mRNA, quantitative RT-PCR, immunologic detection of a DA-like GPCR polypeptide, or measurement of DA-like GPCR activity.

In any of the embodiments described above, any of the pharmaceutical compositions of the invention can be administered in combination with other appropriate therapeutic agents. Selection of the appropriate agents for use in combination therapy can be made by one of ordinary skill in the art, according to conventional pharmaceutical principles. The combination of therapeutic agents can act synergistically to effect the treatment or prevention of the various disorders described above. Using this approach, one may be able to achieve therapeutic efficacy with lower dosages of each agent, thus reducing the potential for adverse side effects.

Any of the therapeutic methods described above can be applied to any subject in need of such therapy, including, for example, mammals such as dogs, cats, cows, horses, rabbits, monkeys, and most preferably, humans.

*Diagnostic Methods*

GPCRs also can be used in diagnostic assays for detecting diseases and abnormalities or susceptibility to diseases and abnormalities related to the presence of mutations in the nucleic acid sequences which encode a GPCR. Such diseases, by way of example, are related to cell transformation, such as tumors and cancers, and various cardiovascular disorders, including hypertension and hypotension, as well as diseases arising from abnormal blood flow, abnormal angiotensin-induced aldosterone secretion, and other abnormal control of fluid and electrolyte homeostasis.

Differences can be determined between the cDNA or genomic sequence encoding a GPCR in individuals afflicted with a disease and in normal individuals. If a mutation is observed in some or all of the afflicted individuals but not in normal individuals, then the mutation is likely to be the causative agent of the disease.

Sequence differences between a reference gene and a gene having mutations can be revealed by the direct DNA sequencing method. In addition, cloned DNA segments can be employed as probes to detect specific DNA segments. The sensitivity of this method is greatly enhanced when combined with PCR. For example, a sequencing primer can be used with a double-stranded PCR product or a single-stranded template molecule generated by a modified PCR. The sequence determination is performed by conventional procedures using radiolabeled nucleotides or by automatic sequencing procedures using fluorescent tags.

Genetic testing based on DNA sequence differences can be carried out by detection of alteration in electrophoretic mobility of DNA fragments in gels with or without denaturing agents. Small sequence deletions and insertions can be visualized, for example, by high resolution gel electrophoresis. DNA fragments of different sequences can be distinguished on denaturing formamide gradient gels in which the mobilities of different DNA fragments are retarded in the gel at different positions according to their specific melting or partial melting temperatures (*see, e.g.,* Myers *et al., Science 230,* 1242, 1985). Sequence changes at specific locations can also be revealed by nuclease protection assays, such as RNase and S 1 protection or the chemical cleavage method (e.g., Cotton *et al., Proc. Natl. Acad. Sci. USA 85,* 4397-4401, 1985). Thus, the detection of a specific DNA sequence can be performed by methods such as hybridization, RNase protection, chemical cleavage, direct DNA sequencing or the use of restriction enzymes and Southern blotting of genomic DNA. In addition to direct methods such as gel-electrophoresis and DNA sequencing, mutations can also be detected by *in situ* analysis.

Altered levels of a GPCR also can be detected in various tissues. Assays used to detect levels of the receptor polypeptides in a body sample, such as blood or a tissue biopsy, derived from a host are well known to those of skill in the art and include radioimmunoassays, competitive binding assays, Western blot analysis, and ELISA assays.

All patents and patent applications cited in this disclosure are expressly incorporated herein by reference. The above disclosure generally describes the present invention. A more complete understanding can be obtained by reference to the following specific examples which are provided for purposes of illustration only and are not intended to limit the scope of the invention.

EXAMPLE 1

*Detection of dopamine-like GPCR activity*

The polynucleotide of SEQ ID NO. 1 is inserted into the expression vector pCEV4 and the expression vector pCEV4-dopamine-like GPCR polypeptide obtained is transfected into human embryonic kidney 293 cells. The cells are scraped from a culture flask into 5 ml of Tris HCl, 5 mM EDTA, pH 7.5, and lysed by sonication. Cell lysates are centrifuged at 1000 rpm for 5 minutes at 4 °C. The supernatant is centrifuged at 30,000 x g for 20 minutes at 4°C. The pellet is suspended in binding buffer containing 50 mM Tris HCl, 5 mM $MgSO_4$, 1 mM EDTA, 100 mM NaCl, pH 7.5, supplemented with 0.1 % BSA, 2 μg/ml aprotinin, 0.5 mg/ml leupeptin, and 10 μg/ml phosphoramidon. Optimal membrane suspension dilutions, defined as the protein concentration required to bind less than 10 % of an added radioligand, i.e. $^{125}$I-labeled dopamine, are added to 96-well polypropylene microtiter plates containing ligand, non-labeled peptides, and binding buffer to a final volume of 250 μl.

In equilibrium saturation binding assays, membrane preparations are incubated in the presence of increasing concentrations (0.1 nM to 4 nM) of $^{125}$I ligand.

Binding reaction mixtures are incubated for one hour at 30°C. The reaction is stopped by filtration through GF/B filters treated with 0.5% polyethyleneimine, using a cell harvester. Radioactivity is measured by scintillation counting, and data are analyzed by a computerized non-linear regression program. Non-specific binding is defined as the amount of radioactivity remaining after incubation of membrane protein in the presence of 100 nM of unlabeled peptide. Protein concentration is measured by the Bradford method using Bio-Rad Reagent, with bovine serum albumin as a standard. The dopamine-like GPCR activity of the polypeptide comprising the amino acid sequence of SEQ ID NO. 2 is demonstrated

EXAMPLE 2

*Radioligand binding assays*

Human embryonic kidney 293 cells transfected with a polynucleotide which expresses human DA-like GPCR are scraped from a culture flask into 5 ml of Tris HCl, 5 mM EDTA, pH 7.5, and lysed by sonication. Cell lysates are centrifuged at 1000 rpm for 5 minutes at 4°C. The supernatant is centrifuged at 30,000 x g for 20 minutes at 4°C. The pellet is suspended in binding buffer containing 50 mM Tris HCl, 5 mM $MgSO_4$, 1 mM EDTA, 100 mM NaCl, pH 7.5, supplemented with 0.1 % BSA, 2 μg/ml aprotinin, 0.5 mg/ml leupeptin, and 10 μg/ml phosphoramidon. Optimal membrane suspension dilutions, defined as the protein concentration required to bind less than 10 % of the added radioligand, i.e. dopamine, are added to 96-well polypropylene microtiter plates containing $^{125}$I-labeled ligand or test compound, non-labeled peptides, and binding buffer to a final volume of 250 μl.

In equilibrium saturation binding assays, membrane preparations are incubated in the presence of increasing concentrations (0.1 nM to 4 nM) of $^{125}$I-labeled ligand or test compound (specific activity 2200 Ci/mmol). The binding affinities of different test compounds are determined in equilibrium competition binding assays, using 0.1 nM $^{125}$I- peptide in the presence of twelve different concentrations of each test compound.

Binding reaction mixtures are incubated for one hour at 30°C. The reaction is stopped by filtration through GF/B filters treated with 0.5% polyethyleneimine, using a cell harvester. Radioactivity is measured by scintillation counting, and data are analyzed by a computerized non-linear regression program.

Non-specific binding is defined as the amount of radioactivity remaining after incubation of membrane protein in the presence of 100 nM of unlabeled peptide. Protein concentration is measured by the Bradford method using Bio-Rad Reagent, with bovine serum albumin as a standard. A test compound which increases the radioactivity of membrane protein by at least 15% relative to radioactivity of membrane protein which was not incubated with a test compound is identified as a compound which binds to a human DA-like GPCR polypeptide.

EXAMPLE 3

*Effect of a test compound on human DA-like GPCR -mediated cyclic AMP formation*

Receptor-mediated inhibition of cAMP formation can be assayed in host cells which express human DA-like GPCR. Cells are plated in 96-well plates and incubated in Dulbecco's phosphate buffered saline (PBS) supplemented with 10 mM HEPES, 5 mM theophylline, 2 μg/ml aprotinin, 0.5 mg/ml leupeptin, and 10 μg/ml phosphoramidon for 20 minutes at 37°C in 5% CO2. A test compound is added and incubated for an additional 10 minutes at 37°C. The medium is aspirated, and the reaction is stopped by the addition of 100 mM HCl. The plates are stored at 4°C for 15 minutes. cAMP content in the stopping solution is measured by radioimmunoassay.

Radioactivity is quantified using a gamma counter equipped with data reduction software. A test compound which decreases radioactivity of the contents of a well relative to radioactivity of the contents of a well in the absence of the test compound is identified as a potential inhibitor of cAMP formation. A test compound which increases radioactivity of the contents of a well relative to radioactivity of the contents of a well in the absence of the test compound is identified as a potential enhancer of cAMP formation.

EXAMPLE 4

*Effect of a test compound on the mobilization of intracellular calcium*

Intracellular free calcium concentration can be measured by microspectrofluorometry using the fluorescent indicator dye Fura-2/AM (Bush *et al., J. Neurochem. 57,* 562-74, 1991). Stably transfected cells are seeded onto a 35 mm culture dish containing a glass coverslip insert. Cells are washed with HBS , incubated with a test compound, and loaded with 100 μl of Fura-2/AM (10 μM) for 20-40 minutes. After washing with HBS to remove the Fura-2/AM solution, cells are equilibrated in HBS for 10-20 minutes. Cells are then visualized under the 40X objective of a Leitz Fluovert FS microscope.

Fluorescence emission is determined at 510 nM, with excitation wavelengths alternating between 340 nM and 380 nM. Raw fluorescence data are converted to calcium concentrations using standard calcium concentration curves and software analysis techniques. A test compound which increases the fluorescence by at least 15% relative to fluorescence in the absence of a test compound is identified as a compound which mobilizes intracellular calcium.

EXAMPLE 5

*Effect of a test compound on phosphoinositide metabolism*

Cells which stably express human DA-like GPCR cDNA are plated in 96-well plates and grown to confluence. The day before the assay, the growth medium is changed to 100 μl of medium containing 1% serum and 0.5 βCi $^3$H-myinositol. The plates are incubated overnight in a $CO_2$ incubator (5% $CO_2$ at 37°C). Immediately before the assay, the medium is removed and replaced by 200 μl of PBS containing 10 mM LiCl, and the cells are equilibrated with the new medium for 20 minutes. During this interval, cells also are equilibrated with antagonist, added as a 10 μl aliquot of a 20-fold concentrated solution in PBS.

The $^3$H-inositol phosphate accumulation from inositol phospholipid metabolism is started by adding 10 μl of a solution containing a test compound. To the first well 10 μl are added to measure basal accumulation. Eleven different concentrations of test compound are assayed in the following 11 wells of each plate row. All assays are performed in duplicate by repeating the same additions in two consecutive plate rows.

The plates are incubated in a $CO_2$ incubator for one hour. The reaction is terminated by adding 15 μl of 50% v/v trichloroacetic acid (TCA), followed by a 40 minute incubation at 4°C. After neutralizing TCA with 40 μl of 1 M Tris, the content of the wells is transferred to a Multiscreen HV filter plate (Millipore) containing Dowex AG1-X8 (200-400 mesh, formate form). The filter plates are prepared by adding 200 μl of Dowex AG1-X8 suspension (50% v/v, water: resin) to each well. The filter plates are placed on a vacuum manifold to wash or elute the resin bed. Each well is washed 2 times with 200 μl of water, followed by 2 x 200 μl of 5 mM sodium tetraborate/60 mM ammonium formate.

The $^3$H-IPs are eluted into empty 96-well plates with 200 μl of 1.2 M ammonium formate/0.1 formic acid. The content of the wells is added to 3 ml of scintillation cocktail, and radioactivity is determined by liquid scintillation counting.

EXAMPLE 6

*Receptor Binding Methods*

Standard Binding Assays. Binding assays are carried out in a binding buffer containing 50 mM HEPES, pH 7.4, 0.5% BSA, and 5 mM $MgCl_2$. The standard assay for radioligand binding to membrane fragments comprising DA-like GPCR polypeptides is carried out as follows in 96 well microtiter plates (*e.g.*, Dynatech Immulon II Removawell plates). Radioligand is diluted in binding buffer+ PMSF/Baci to the desired cpm per 50 μl, then 50 μl aliquots are added to the wells. For non-specific binding samples, 5 μl of 40 μM cold ligand also is added per well. Binding is initiated by adding 150 μl per well of membrane diluted to the desired concentration (10-30 μg membrane protein/well) in binding buffer+ PMSF/Baci. Plates are then covered with Linbro mylar plate sealers (Flow Labs) and placed on a Dynatech Microshaker II. Binding is allowed to proceed at room temperature for 1-2 hours and is stopped by centrifuging the plate for 15 minutes at 2,000 x g. The supernatants are decanted, and the membrane pellets are washed once by addition of 200 μl of ice cold binding buffer, brief shaking, and recentrifugation. The individual wells are placed in 12 x 75 mm tubes and counted in an LKB Gammamaster counter (78% efficiency). Specific binding by this method is identical to that measured when free ligand is removed by rapid (3-5 seconds) filtration and washing on polyethyleneimine-coated glass fiber filters.

Three variations of the standard binding assay are also used.

1. Competitive radioligand binding assays with a concentration range of cold ligand vs. $^{125}$ I-labeled ligand are carried out as described above with one modification. All dilutions of ligands being assayed are made in 40X PMSF/Baci to a concentration 40X the final concentration in the assay. Samples of peptide (5 μl each) are then added per microtiter well. Membranes and radioligand are diluted in binding buffer without protease inhibitors. Radioligand is added and mixed with cold ligand, and then binding is initiated by addition of membranes.

2. Chemical cross-linking of radioligand with receptor is done after a binding step identical to the standard assay. However, the wash step is done with binding buffer minus BSA to reduce the possibility of non-specific cross-linking of radioligand with BSA. The cross-linking step is carried out as described below.

3. Larger scale binding assays to obtain membrane pellets for studies on solubilization of receptor:ligand complex and for receptor purification are also carried out. These are identical to the standard assays except that (a) binding is carried out in polypropylene tubes in volumes from 1-250 ml, (b) concentration of membrane protein is always 0.5 mg/ml, and (c) for receptor purification, BSA concentration in the binding buffer is reduced to 0.25%, and the wash step is done with binding buffer without BSA, which reduces BSA contamination of the purified receptor.

EXAMPLE 7

*Chemical Cross-Linking ofRadioligand to Receptor*

After a radioligand binding step as described above, membrane pellets are resuspended in 200 μl per microtiter plate well of ice-cold binding buffer without BSA. Then 5 μl per well of 4 mM N-5-azido-2-nitrobenzoyloxysuccinimide (ANB-NOS, Pierce) in DMSO is added and mixed. The samples are held on ice and UV-irradiated for 10 minutes with a Mineralight R-52G lamp (UVP Inc., San Gabriel, Calif.) at a distance of 5-10 cm. Then the samples are transferred to Eppendorf microfuge tubes, the membranes pelleted by centrifugation, supernatants removed, and membranes solubilized in Laemmli SDS sample buffer for polyacrylamide gel electrophoresis (PAGE). PAGE is carried out as described below. Radiolabeled proteins are visualized by autoradiography of the dried gels with Kodak XAR film and DuPont image intensifier screens.

EXAMPLE 8

*Membrane Solubilization*

Membrane solubilization is carried out in buffer containing 25 mM Tris , pH 8, 10% glycerol (w/v) and 0.2 mM $CaCl_2$ (solubilization buffer). The highly soluble detergents including Triton X-100, deoxycholate, deoxycholate:lysolecithin, CHAPS, and zwittergent are made up in solubilization buffer at 10% concentrations and stored as frozen aliquots. Lysolecithin is made up fresh because of insolubility upon freeze-thawing and digitonin is made fresh at lower concentrations due to its more limited solubility.

To solubilize membranes, washed pellets after the binding step are resuspended free of visible particles by pipetting and vortexing in solubilization buffer at 100,000 x g for 30 minutes. The supernatants are removed and held on ice and the pellets are discarded.

EXAMPLE 9

*Assay of Solubilized Receptors*

After binding of $^{125}$I ligands and solubilization of the membranes with detergent, the intact R:L complex can be assayed by four different methods. All are carried out on ice or in a cold room at 4-10°C.).

1. Column chromatography (Knuhtsen *et al., Biochem. J. 254*, 641-647, 1988). Sephadex G-50 columns (8 x 250 mm) are equilibrated with solubilization buffer containing detergent at the concentration used to solubilize membranes and 1 mg/ml bovine serum albumin. Samples of solubilized membranes (0.2-0.5 ml) are applied to the columns and eluted at a flow rate of about 0.7 ml/minute. Samples (0.18 ml) are collected. Radioactivity is determined in a gamma counter. Void volumes of the columns are determined by the elution volume of blue dextran. Radioactivity eluting in the void volume is considered bound to protein. Radioactivity eluting later, at the same volume as free $^{125}$I ligands, is considered non-bound.

2. Polyethyleneglycol precipitation (Cuatrecasas, *Proc. Natl. Acad. Sci. USA 69,* 318-322, 1972). For a 100 μl sample of solubilized membranes in a 12 x 75 mm polypropylene tube, 0.5 ml of 1% (w/v) bovine gamma globulin (Sigma) in 0.1 M sodium phosphate buffer is added, followed by 0.5 ml of 25% (w/v) polyethyleneglycol (Sigma) and mixing. The mixture is held on ice for 15 minutes. Then 3 ml of 0.1 M sodium phosphate, pH 7.4, is added per sample. The samples are rapidly (1-3 seconds) filtered over Whatman GF/B glass fiber filters and washed with 4 ml of the phosphate buffer. PEG-precipitated receptor : $^{125}$ I-ligand complex is determined by gamma counting of the filters.

3. GFB/PEI filter binding (Bruns *et al., Analytical Biochem. 132,* 74-81, 1983). Whatman GF/B glass fiber filters are soaked in 0.3% polyethyleneimine (PEI, Sigma) for 3 hours. Samples of solubilized membranes (25-100 μl) are replaced in 12 x 75 mm polypropylene tubes. Then 4 ml of solubilization buffer without detergent is added per sample and the samples are immediately filtered through the GFB/PEI filters (1-3 seconds) and washed with 4 ml of solubilization buffer. CPM of receptor : $^{125}$ I-ligand complex adsorbed to filters are determined by gamma counting.

4. Charcoal/Dextran (Paul and Said, *Peptides 7[Suppl. 1]*,147-149, 1986). Dextran T70 (0.5 g, Pharmacia) is dissolved in 1 liter of water, then 5 g of activated charcoal (Norit A, alkaline; Fisher Scientific) is added. The suspension is stirred for 10 minutes at room temperature and then stored at 4°C. until use. To measure R:L complex, 4 parts by volume of charcoal/dextran suspension are added to 1 part by volume of solubilized membrane. The samples are mixed and held on ice for 2 minutes and then centrifuged for 2 minutes at 11,000 x g in a Beckman microfuge. Free radioligand is adsorbed charcoal/dextran and is discarded with the pellet. Receptor : $^{125}$I-ligand complexes remain in the supernatant and are determined by gamma counting.

EXAMPLE 10

*Receptor Purification*

Binding of biotinyl-receptor to $GH_4$ C1 membranes is carried out as described above. Incubations are for 1 hour at room temperature. In the standard purification protocol, the binding incubations contain 10 nM Bio-S29. $^{125}$I ligand is added as a tracer at levels of 5,000-100,000 cpm per mg of membrane protein. Control incubations contain 10 μM cold ligand to saturate the receptor with non-biotinylated ligand.

Solubilization of receptor:ligand complex also is carried out as described above, with 0.15% deoxycholate: lysolecithin in solubilization buffer containing 0.2 mM $MgCl_2$, to obtain 100,000 x g supernatants containing solubilized R:L complex.

Immobilized streptavidin (streptavidin cross-linked to 6% beaded agarose, Pierce Chemical Co.; "SA-agarose") is washed in solubilization buffer and added to the solubilized membranes as 1/30 of the final volume. This mixture is incubated with constant stirring by end-over-end rotation for 4-5 hours at 4-10 °C. Then the mixture is applied to a column and the non-bound material is washed through. Binding of radioligand to SA-agarose is determined by comparing cpm in the 100,000 x g supernatant with that in the column effluent after adsorption to SA-agarose. Finally, the column is washed with 12-15 column volumes of solubilization buffer+0.15% deoxycholate:lysolecithin +1/500 (vol/vol) 100 x 4pase.

The streptavidin column is eluted with solubilization buffer+0.1 mM EDTA+0.1 mM EGTA+0.1 mM GTP-gamma-S (Sigma)+0.15% (wt/vol) deoxycholate:lysolecithin +1/1000 (vol/vol) 100.times.4pase. First, one column volume of elution buffer is passed through the column and flow is stopped for 20-30 minutes. Then 3-4 more column volumes of elution buffer are passed through. All the eluates are pooled.

Eluates from the streptavidin column are incubated overnight (12-15 hours) with immobilized wheat germ agglutinin (WGA agarose, Vector Labs) to adsorb the receptor via interaction of covalently bound carbohydrate with the WGA lectin. The ratio (vol/vol) of WGA-agarose to streptavidin column eluate is generally 1:400. A range from 1:1000 to 1:200 also can be used. After the binding step, the resin is pelleted by centrifugation, the supernatant is removed and saved, and the resin is washed 3 times (about 2 minutes each) in buffer containing 50 mM HEPES, pH 8, 5 mM $MgCl_2$, and 0.15% deoxycholate:lysolecithin. To elute the WGA-bound receptor, the resin is extracted three times by repeated mixing (vortex mixer on low speed) over a 15-30 minute period on ice, with 3 resin columns each time, of 10 mM N-N'-N"-triacetylchitotriose in the same HEPES buffer used to wash the resin. After each elution step, the resin is centrifuged down and the supernatant is carefully removed, free of WGA-agarose pellets. The three, pooled eluates contain the final, purified receptor. The material non-bound to WGA contain G protein subunits specifically eluted from the streptavidin column, as well as non-specific contaminants. All these fractions are stored frozen at -90°C.

EXAMPLE 11

*Identification of test compounds that bind to DA-like GPCR polypeptides*

Purified DA-GPCR polypeptides comprising a glutathione-S-transferase protein and absorbed onto glutathione-derivatized wells of 96-well microtiter plates are contacted with test compounds from a small molecule library at pH 7.0 in a physiological buffer solution. DA-GPCR polypeptides comprise an amino acid sequence shown in SEQ ID NO. 2. The test compounds comprise a fluorescent tag. The samples are incubated for 5 minutes to one hour. Control samples are incubated in the absence of a test compound.

The buffer solution containing the test compounds is washed from the wells. Binding of a test compound to a DA-like GPCR polypeptide is detected by fluorescence measurements of the contents of the wells. A test compound which increases the fluorescence in a well by at least 15% relative to fluorescence of a well in which a test compound is not incubated is identified as a compound which binds to a DA-like GPCR polypeptide.

EXAMPLE 12

*Identification of a test compound which decreases DA-like GPCR gene expression*

A test compound is administered to a culture of human gastric cells and incubated at 37°C for 10 to 45 minutes. A culture of the same type of cells incubated for the same time without the test compound provides a negative control.

RNA is isolated from the two cultures as described in Chirgwin *et al., Biochem. 18,* 5294-99, 1979). Northern blots are prepared using 20 to 30 μg total RNA and hybridized with a $^{32}$P-labeled DA-like GPCR-specific probe at 65° C in Express-hyb (CLONTECH). The probe comprises at least 11 contiguous nucleotides selected from the complement of SEQ ID NO. 1. A test compound which decreases the DA-like GPCR- specific signal relative to the signal obtained in the absence of the test compound is identified as an inhibitor of DA-like GPCR gene expression.

EXAMPLE 13

*Treatment of schizophrenia with a reagent which specifically binds to a DA-like GPCR gene product*

Synthesis of antisense DA-like GPCR oligonucleotides comprising at least 11 contiguous nucleotides selected from the complement of SEQ ID NO. 1 is performed on a Pharmacia Gene Assembler series synthesizer using the phosphoramidite procedure (Uhlmann *et al., Chem. Rev. 90,* 534-83, 1990). Following assembly and deprotection, oligonucleotides are ethanol-precipitated twice, dried, and suspended in phosphate-buffered saline (PBS) at the desired concentration. Purity of these oligonucleotides is tested by capillary gel electrophoreses and ion exchange HPLC. Endotoxin levels in the oligonucleotide preparation are determined using the *Limulus* Amebocyte Assay (Bang, *Biol. Bull.* (*Woods Hole, Mass.) 105*, 361-362, 1953).

The antisense oligonucleotides are administered to a patient with schizophrenia. The severity of the patient's schizophrenia is lessened.

EXAMPLE 14

*Ligand Binding Analysis*

COS-7 cells are harvested 48 to 72 hr after transfection with human DA-like GPCR expression constructs. Cells contained in culture flasks (75 $cm^2$) are rinsed with 5 ml of lysis buffer (10 mM Tris-HCl, 5 mM EDTA, pH 7.4). Cells are then scraped and homogenized in lysis buffer for 15 seconds using a Brinkman homogenizer.

Membranes are centrifuged at 50,000 x g for 20 minutes, and the pellet is resuspended in binding buffer (50 mM Tris HCl, 100 mM NaCl, 5 mM EDTA, pH 7.4). Saturation binding studies are performed with increasing concentrations of [$^{125}$I]SCH 23982 (2200 Ci/mmol; 1 Ci=37 GBq). Competition curves are performed with increasing concentrations of unlabeled drug under study against a constant concentration of [$^{125}$I]SCH 233982 (approximately 0.20 nM). The reaction is initiated by adding 100 μl of membranes (approximately 0.45 μg protein), and the assay mixture is incubated in a final volume of 200 μl for 1 hour at room temperature. Assay mixtures are then vacuum-filtered through Whatman GF/C glassfiber filters and washed 3 times with 5 ml of cold washing buffer (50 mM Tris HCl, 100 mM NaCl, pH 7.2). Total and nonspecific binding are delineated in the absence and presence of 10 μM cis-flupentixol. Each determination is done in triplicate. Bound radioactivity is measured at an efficiency of 75% using a gamma counter (LKB instruments). Binding curves are analyzed using non-linear multiple regression programs. See DeLean *et al., Mol. Pharmacol. 21,* 5-16 (1982); McPherson, *J. Pharmacol. Methods 14,* 213-228 (1985).

Cis-flupentixol, cis-piflutixol, cis-teflutixol can be obtained from Lundbeck (Denmark). Fluperlapine can be obtained from Novo Nordisk (Bagsvaerd, Denmark). SCH 23388 AND SCH 23390 can be obtained from Schering Plough (Bloomfield, N.J.). Apomorphine, (+)butaclamol, dopamine hydrochloride, haloperidol, R(-)- propylnorapomorphine (NPA) and spiperone can be purchased from Research Biochemical Industries (RBI). Fenoldopam and SKF 38393 can be obtained from Smith, Kline & French. [$^{125}$ I]SCH 23982 was from New England Nuclear Boston, Mass.).

EXAMPLE 15

*Tissue-specific expression of Dopamine-like GPCR*

The expression profiling of the Dopamine-like GPCR by real-time quantitative PCR with RNA samples from human tissue establishes a role for the Dopamine-like GPCR in human disease, The panel consists of total RNA from brain, total Alzheimer brain, fetal brain, cerebellum, spinal cord, heart, fetal heart, pericardium, coronary smooth muscle cells, thyroid, thyroid tumor, spleen, spleen (from liver cirrhosis patient), thymus, bone marrow, lung, fetal lung, lung tumor, liver, fetal liver, liver (from liver cirrhosis patient), pancreas, pancreas (from liver cirrhosis patient), stomach, small intestine, colon, colon tumor, kidney, skeletal muscle, HeLa cells, breast tumor, mammary gland, MDA MB 231 cells, testis, trachea, adrenal gland, salivary gland, bladder, prostata, placenta, uterus, adipose tissue. A development of the kinetic analysis of PCR is described in Higuchi *et al., BioTechnology 10, 413-17,* 1992, and Higuchi *et al., BioTechnology 11,* 1026-30, 1993. The principle is that at any given cycle within the exponential phase of PCR, the amount of product is proportional to the initial number of template copies.

PCR amplification is performed in the presence of an oligonucleotide probe (TaqMan probe) that is complementary to the target sequence and labeled with a fluorescent reporter dye and a quencher dye. During the extension phase of PCR, the probe is cleaved by the 5'-3' endonuclease activity of Taq DNA polymerase, releasing the fluorophore from the effect of the quenching dye (Holland *et al., Proc. Natl. Acad. Sci. U.S.A. 88,* 7276-80, 1991). Because the fluorescence emission increases in direct proportion to the amount of the specific amplified product, the exponential growth phase of PCR product can be detected and used to determine the initial template concentration (Heid *et al., Genome Res. 6*, 986-94, 1996, and Gibson *et al., Genome Res. 6*, 995-1001, 1996).

Real-time quantitative PCR is done using an ABI Prism 7700 Sequence Detector.

The $C_T$ value generated for each reaciton is used to determine the initial template concentration (copy number) by interpolation from a universal standard curve. The level of expression of the target gene in each sample is calculated relative to the sample with the lowest expression of the gene.

*RNA extraction and cDNA preparation.* Total RNA from each of the cell types listed above were isolated using Qiagen's RNeasy system according to the manufacturer's protocol (Crawley, West Sussex, UK). The concentration of purified RNA is determined using a RiboGreen RNA quantitation kit (Molecular Probes Europe, The Netherlands). For the preparation of cDNA, 1 $\mu$g of total RNA is reverse transcribed in a final volume of 20 $\mu$l, using 200 U of SUPERSCRIPT™ RNase $H^-$ Reverse Transcriptase (Life Technologies, Paisley, UK), 10 mM dithiothreitol, 0.5 mM of each dNTP and 5 $\mu$M random hexamers (Applied Biosystems, Warrington, Cheshire, UK) according to the manufacturer's protocol.

*TaqMan quantitative analysis.* Specific primers and probe were designed according to the recommendations of PE Applied Biosystems; a FAM (6-carboxy-fluorescein)-labeled probe is used. Quantification PCR is performed with 5 ng of reverse transcribed RNA from each sample. Each determination is done in duplicate.

The assay reaction mix is as follows: 1X final TaqMan Universal PCR Master Mix (from 2X stock) (PE Applied Biosystems, CA); 900 nM forward primer; 900 nM reverse primer; 200 nM probe; 5 ng cDNA; and water to 25 $\mu$l.

Each of the following steps were carried out once: pre PCR, 2 minutes at 50°C, and 10 minutes at 95°C. The following steps are carried out 40 times: denaturation, 15 seconds at 95°C, annealing/extension, 1 minute at 60°C.

All experiments were performed using an ABI Prism 7700 Sequence Detector (PE Applied Biosystems, CA). At the end of the run, fluorescence data acquired during PCR were processed as described in the ABI Prism 7700 user's manual to achieve better background subtraction as well as signal linearity with the starting target quantity.

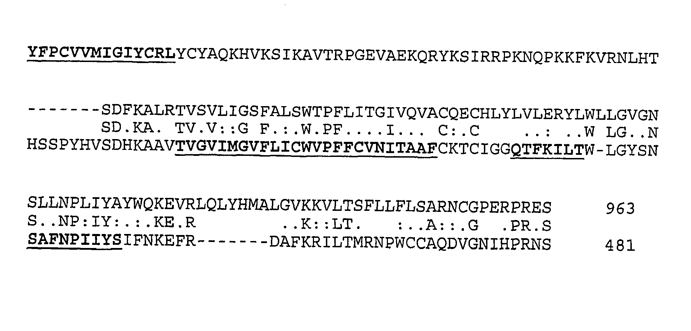

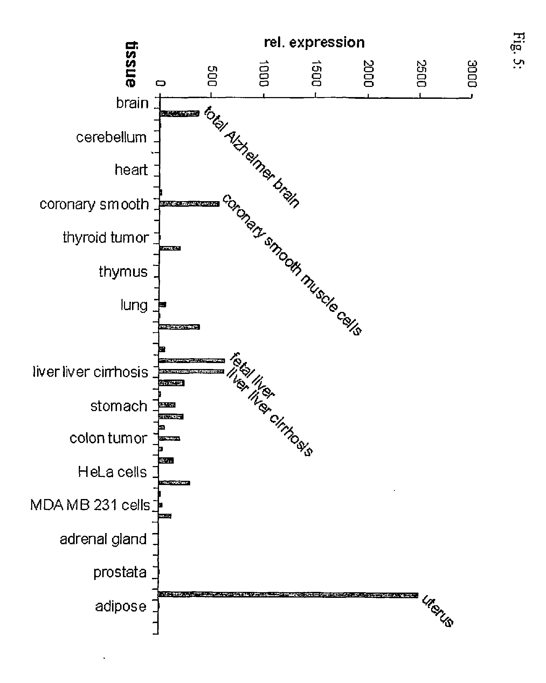

Table 1 shows the results of expression profiling for Dopamine-like GPCR using the indicated cell and tissue samples. The result is shown graphically in Fig. 5. Relative high expression is detected in Alzheimer's brain compared to normal brain and in cancer tissue compared to the corresponding normal tissue.

Table 1:

| | tissue | rel ex | | tissue | rel ex |
|---|---|---|---|---|---|
| 1. | brain | 0,00 | 22. | pancreas | 240,57 |
| 2. | total Alzheimer brain | 379,60 | 23. | pancreas liver cirrhosis | 14,47 |
| 3. | fetal brain | 13,39 | 24. | stomach | 160,85 |
| 4. | cerebellum | 0,00 | 25. | small intestine | 234,24 |
| 5. | spinal cord | 2,52 | 26. | colon | 56,98 |
| 6. | heart | 0,00 | 27. | colon tumor | 200,85 |
| 7. | fetal heart | 1,14 | 28. | kidney | 38,63 |
| 8. | pericardium | 16,24 | 29. | skeletal muscle | 144,79 |
| 9. | coronary smooth muscle cells | 578,70 | 30. | HeLa cells | 1,96 |
| 10. | thyroid | 0,00 | 31. | breast tumor | 300,25 |
| 11. | thyroid tumor | 7,34 | 32. | mammary gland | 17,44 |
| 12. | spleen | 199,84 | 33. | MDA MB 231 cells | 34,54 |

(continued)

| | tissue | rel ex | | tissue | rel ex |
|---|---|---|---|---|---|
| 13. | spleen liver cirrhosis | 1,00 | 34. | testis | 124,01 |
| 14. | thymus | 0,00 | 35. | trachea | 0,00 |
| 15. | bone marrow | 0,00 | 36. | adrenal gland | 0,00 |
| 16. | lung | 62,50 | 37. | salivary gland | 0,00 |
| 17. | fetal lung | 10,18 | 38. | bladder | 0,00 |
| 18. | lung tumor | 393,89 | 39. | prostata | 10,37 |
| 19. | liver | 60,83 | 40. | placenta | 0,00 |
| 20. | fetal liver | 636,96 | 41. | uterus | 2499,15 |
| 21. | liver liver cirrhosis | 628,89 | 42. | adipose | 11,00 |

## Claims

1. A method of screening for agents useful in the treatment of Alzheimer's disease which regulate the activity of a dopamine-like GPCR polypeptide comprising

   (a) contacting a test compound with a dopamine-like GPCR polypeptide comprising

      i. an amino acid sequence comprising SEQ ID NO:2, or
      ii. an amino acid sequence which is at least 90% identical to SEQ ID NO:2;

   and
   (b) detecting a dopamine-like GPCR activity of the polypeptide, wherein a test compound which decreases the dopamine-like GPCR activity is identified as a potential therapeutic agent for decreasing the activity of the dopamine-like GPCR polypeptide.

## Patentansprüche

1. Verfahren zum Screenen auf Mittel, die zur Behandlung der Alzheimer-Krankheit zweckdienlich sind und die Aktivität eines dopaminähnlichen GPCR-Polypeptids regeln, umfassend:

   (a) das Kontaktieren einer Testverbindung mit einem dopaminähnlichen GPCR-Polypeptid, umfassend:

      i. eine Aminosäuresequenz, die Seq.-ID Nr. 2 umfasst, oder
      ii. eine Aminosäuresequenz, die zumindest 90 % Identität mit Seq.-ID Nr. 2 aufweist;

   und
   (b) das Detektieren einer dopaminähnlichen GPCR-Aktivität des Polypeptids, worin eine Testverbindung, welche die dopaminähnliche GPCR-Aktivität verringert, als mögliches Therapeutikum zur Verringerung der Aktivität des dopaminähnlichen GPCR-Polypeptids identifiziert wird.

## Revendications

1. Méthode de criblage pour la détection d'agents utiles dans le traitement de la maladie d'Alzheimer qui régulent l'activité d'un polypeptide GPCR semblable à la dopamine, comprenant

   (a) la mise en contact d'un composé à tester avec un polypeptide GPCR semblable à la dopamine comprenant

i. une séquence d'acides aminés comprenant SEQ ID NO :2, ou
ii. une séquence d'acides aminés qui est au moins à 90 % identique à SEQ ID NO :2 ;

et
(b) la détection d'une activité de GPCR semblable à la dopamine du polypeptide, où un composé testé qui abaisse l'activité de GPCR semblable à la dopamine est identifiée comme un agent thérapeutique potentiel capable d'abaisser l'activité du polypeptide GPCR semblable à la dopamine.

Fig. 1

atggaatcat ctttctcatt tggagtgatc cttgctgtcc tggcctccct
catcattgct actaacacac tagtggctgt ggctgtgctg ctgttgatcc
acaagaatga tggtgtcagt ctctgcttca ccttgaatct ggctgtggct
gacaccttga ttggtgtggc catctctggc ctactcacag accagctctc
cagcccttct cggcccacac agaagaccct gtgcagcctg cggatggcat
ttgtcacttc ctccgcagct gcctctgtcc tcacggtcat gctgatcacc
tttgacaggt accttgccat caagcagccc ttccgctact tgaagatcat
gagtgggttc gtggccgggg cctgcattgc cgggctgtgg ttagtgtctt
acctcattgg cttcctccca ctcggaatcc ccatgttcca gcagactgcc
tacaaagggc agtgcagctt ctttgctgta tttcaccctc acttcgtgct
gaccctctcc tgcgttggct tcttcccagc catgctcctc tttgtcttct
tctactgcga catgctcaag attgcctcca tgcacagcca gcagattcga
aagatggaac atgcaggagc catggctgga ggttatcgat ccccacggac
tcccagcgac ttcaaagctc tccgtactgt gtctgttctc attgggagct
ttgctctatc ctggaccccc ttccttatca ctggcattgt gcaggtggcc
tgccaggagt gtcacctcta cctagtgctg gaacggtacc tgtggctgct
cggcgtgggc aactccctgc tcaacccact catctatgcc tattggcaga
aggaggtgcg actgcagctc taccacatgg ccctaggagt gaagaaggtg
ctcacctcat tcctcctctt tctctcggcc aggaattgtg gcccagagag
gcccagggaa agttcctgtc acatcgtcac tatctccagc tcagagtttg
atggctaa

Fig. 2

MESSFSFGVILAVLASLIIATNTLVAVAVLLLIHKNDGVSLCFTLNLAVA
DTLIGVAISGLLTDQLSSPSRPTQKTLCSLRMAFVTSSAAASVLTVMLIT
FDRYLAIKQPFRYLKIMSGFVAGACIAGLWLVSYLIGFLPLGIPMFQQTA
YKGQCSFFAVFHPHFVLTLSCVGFFPAMLLFVFFYCDMLKIASMHSQQIR
KMEHAGAMAGGYRSPRTPSDFKALRTVSVLIGSFALSWTPFLITGIVQVA
CQECHLYLVLERYLWLLGVGNSLLNPLIYAYWQKEVRLQLYHMALGVKKV
LTSFLLFLSARNCGPERPRESSCHIVTISSSEFDG*

Fig. 3

MYTPHPFGFL IILVPMTNAM RAIAAIAAGV GSVAATVATS TTSSISSSTT
IINTSSATTI GGNHTSGSTG FSTNSTLLDA DHLPLQLTTA KVDLDIEIDI
QLLTNGYDGT TLTSFYNESS WTNASEMDTI VGEEPEPLSL VSIVVVGIFL
SVLIFLSVAG NILVCLAIYT DGSLPRIGNL FLASLAIADL FVASLVMTFA
GVNDLLGYWI FGAQFCDTWV AFDVMCSTAS ILNLCAISMD RYIHIKDPLR
YGRWVTRRVA VITIAAIWLL AAFVSFVPIS LGIHRPDQPL IFEDNGKKYP
TCALDLTPTY AVVSSCISFY FPCVVMIGIY CRLYCYAQKH VKSIKAVTRP
GEVAEKQRYK SIRRPKNQPK KFKVRNLHTH SSPYHVSDHK AAVTVGVIMG
VFLICWVPFF CVNITAAFCK SDRFITDYAA KNVVVMNSGR SSAELEQVSA I

Fig. 4

```
BLASTX - alignment of 115 against swiss|P41596|DOP1_DROME

DOPAMINE RECEPTOR 1 PRECURSOR (D-DOP1).//:trembl|X77234|DMDOPREC_1 product:
"dopamine receptor"; D.melanogaster mRNA

for dopamine receptor //:gp|X77234|479128 product: "dopamine receptor";
D.melanogaster mRNA for dopamine receptor.

     This hit is scoring at : 9e-25 (expectation value)
     Alignment length (overlap) : 350
     Identities : 30 %
     Scoring matrix : BLOSUM62 (used to infer consensus pattern)
     Query reading frame : +1
     Database searched : nrdb

Q:    22 GVILAVLASLIIATNTLVAVAVLLLIHKNDG----VSLCFTLNLAVADTLIGVAISGL-
         G:.L:VL. L :A N.LV.:A:.      .DG    :. .F..:LA:AD..:. .:. .
H:   147 GIFLSVLIFLSVAGNILVCLAIY----TDGSLPRIGNLFLASLAIADLFVASLVMTFAG

        LTDQLSSPSRPTQKTLCSLRMAFVTSSAAASVLTVMLITFDRYLAIKQPFRYLKIMSGFV
        :.D L.     .Q  .C.. :AF ...:.AS:L.:..I:.DRY: IK.P.RY : ::  V
        VNDLLGYWIFGAQùFCDTWVAFDVMCSTASILNLCAISMDRYIHIKDPLRYGRWVTRRV

        AGACIAGLWLVSYLIGFLPLGI-------PMFQQTAYKGQCSFFAVFHPHFVLTLSCVGF
        A ..IA.:WL:: .:.F:P:.:       P:. :.  K . :   . . P :.:. SC:.F
        AVITIAAIWLLAAFVSFVPISLGIHRPDQPLIFEDNGKKYPTCALDLTPTYAVVSSCISF

        FPAMLLFVFFYCDMLKIASMHSQQIRKMEHAGAMA--GGYRSPRTP-------------
        : ..:::.: .YC :. .A..H :.I:.:...G.:A    Y:S R.P
```

```
YFPCVVMIGIYCRLYCYAQKHVKSIKAVTRPGEVAEKQRYKSIRRPKNQPKKFKVRNLHT

-------SDFKALRTVSVLIGSFALSWTPFLITGIVQVACQECHLYLVLERYLWLLGVGN
       SD.KA. TV.V::G F.:.W.PF....I... C:.C     ..: ..W LG..N
HSSPYHVSDHKAAVTVGVIMGVFLICWVPFFCVNITAAFCKTCIGGQTFKILTW-LGYSN

SLLNPLIYAYWQKEVRLQLYHMALGVKKVLTSFLLFLSARNCGPERPRES     963
S..NP:IY:.:.KE.R        ..K::LT.   :..A::.G  .PR.S
SAFNPIIYSIFNKEFR-------DAFKRILTMRNPWCCAQDVGNIHPRNS     481
```

Fig. 5:

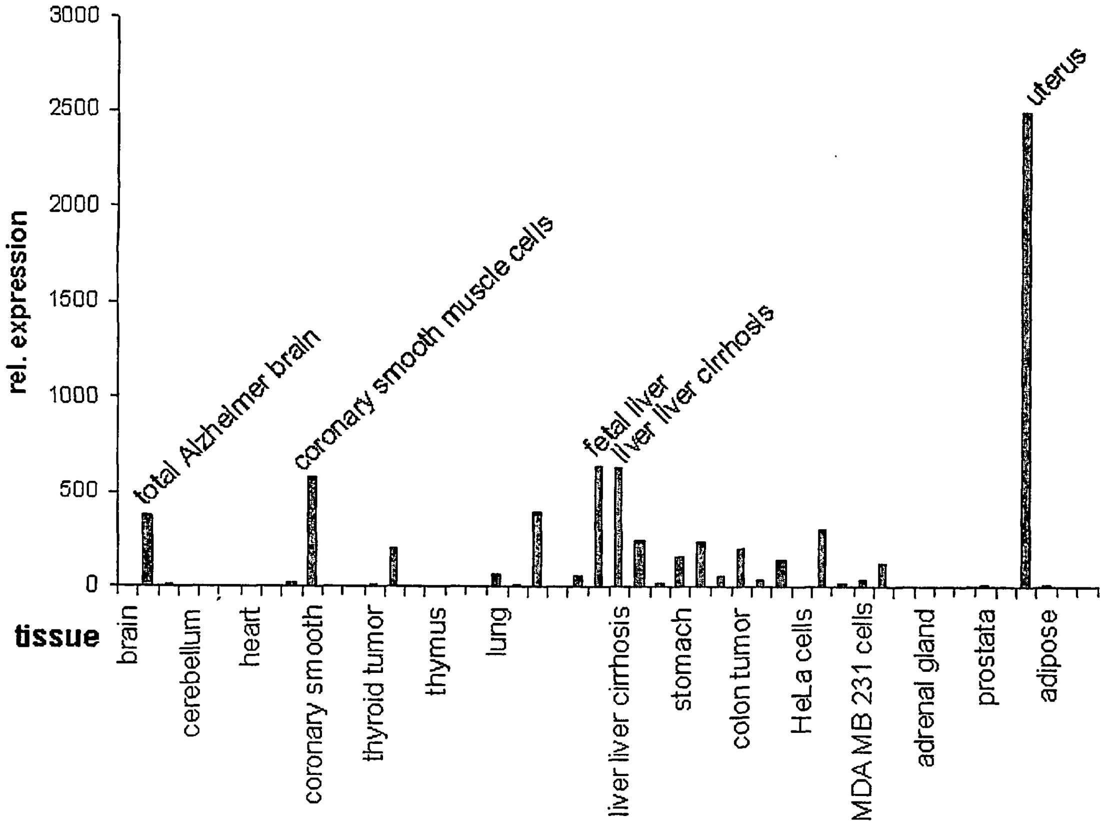
rel. expression
3000
2500
2000
1500
1000
500
0
total Alzheimer brain
coronary smooth muscle cells
fetal liver
liver liver cirrhosis
uterus
tissue
brain
cerebellum
heart
coronary smooth
thyroid tumor
thymus
lung
liver liver cirrhosis
stomach
colon tumor
HeLa cells
MDA MB 231 cells
adrenal gland
prostata
adipose